(19) **Europäisches Patentamt / European Patent Office / Office européen des brevets**

(11) **EP 3 510 051 B2**

(12) **NOUVEAU FASCICULE DE BREVET EUROPEEN**
Après la procédure d'opposition

(45) Date de publication et mention de la décision concernant l'opposition:
**05.10.2022 Bulletin 2022/40**

(45) Mention de la délivrance du brevet:
**01.01.2020 Bulletin 2020/01**

(21) Numéro de dépôt: **18799773.9**

(22) Date de dépôt: **09.11.2018**

(51) Classification Internationale des Brevets (IPC):
**C08B 37/08** (2006.01)

(52) Classification Coopérative des Brevets (CPC):
(C-Sets disponibles)
**C08B 37/003; A61K 8/736; A61K 31/722; A61L 27/20; A61Q 19/08; C08L 5/08;**
A61L 2400/06 (Cont.)

(86) Numéro de dépôt international:
**PCT/EP2018/080767**

(87) Numéro de publication internationale:
**WO 2019/105719 (06.06.2019 Gazette 2019/23)**

(54) **CARBOXYALKYL CHITOSANE**

CARBOXYALKYL CHITOSANE

CARBOXYALKYL CHITOSAN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.11.2017 FR 1761323**

(43) Date de publication de la demande:
**17.07.2019 Bulletin 2019/29**

(73) Titulaire: **Kiomed Pharma**
**4040 Herstal (BE)**

(72) Inventeurs:
• **CHAUSSON, Mickael**
**4500 Huy (BE)**
• **DOUETTE, Pierre**
**4053 Embourg (BE)**
• **GAUTIER, Sandrine, Emilia**
**4000 Liege (BE)**
• **VAESEN, Philippe**
**4672 SAINT REMY (BE)**
• **CHOUMANE, Houtai**
**4053 Embourg (BE)**
• **ROCASALBAS, Guillermo**
**4000 Liege (BE)**

(74) Mandataire: **Portal, Frédéric**
**Bird & Bird AARPI**
**2, rue de la Chaussée d'Antin**
**75009 Paris (FR)**

(56) Documents cités:
**EP-A1- 3 156 059**

• Y.F. POON ET AL: "Cytocompatible Hydrogels Based on Photocrosslinkable Methacrylated O-Carboxymethylchitosan with Tunable Charge: Synthesis and Characterization", ADVANCED FUNCTIONAL MATERIALS, vol. 17, no. 13, 3 septembre 2007 (2007-09-03), pages 2139-2150, XP055482200, DE ISSN: 1616-301X, DOI: 10.1002/adfm.200600420
• DI MARIO F ET AL: "Chitin and chitosan from Basidiomycetes", INTERNATIONAL JOURNAL OF BIOLOGICAL MACROMOLECULES, ELSEVIER BV, NL, vol. 43, no. 1, 1 juillet 2008 (2008-07-01), pages 8-12, XP022705839, ISSN: 0141-8130, DOI: 10.1016/J.IJBIOMAC.2007.10.005 [extrait le 2008-06-05]
• UPADHYAYA L. et al.: "Biomedical applications of carboxymethyl chitosans", Carbohydrate Polymers, vol. 91, no. 1, 2013, pages 452-466,
• Zamam Akam, Suprabsorbent Polymers form the cell wall, of Zygomycetes Funghi Skrifter fran Hogskolan Boras Vol. 23, 2010, ISSN:- 978-91-7385-417-7

(52) Classification Coopérative des Brevets (CPC):
(Cont.)

C-Sets
**A61L 27/20, C08L 5/08**

(52) Classification Coopérative des Brevets (CPC):
(Cont.)

C-Sets
**A61L 27/20, C08L 5/08**

**Description**

La présente invention concerne un carboxyalkyl chitosane, des compositions le comprenant, son procédé de fabrication et ses différentes applications, en particulier dans le domaine thérapeutique, rhumatologique, ophtalmologique, médecine esthétique, chirurgie plastique, chirurgie interne, dermatologique, ou cosmétique.

**Etat de la technique**

Il est connu des dérivés de chitosane, notamment dans les demandes de Kiomed Pharma publiées sous les numéros WO 2016/016463 et WO 2016/016464 et les brevets correspondants. Ces demandes se concentrent sur les propriétés physiques, chimiques ou physico-chimiques des dérivés de chitosane. Ces compositions demandent encore à être améliorées, en particulier dans le cadre d'un traitement thérapeutique de manière à procurer aux patients qui peuvent être amenés à utiliser de telles compositions un bénéfice thérapeutique optimisé, et en particulier à augmenter le rapport bénéfice/risque.

On peut obtenir un chitosane soluble par augmentation du degré d'acétylation (DA) par réacétylation du chitosane fongique. On peut en effet obtenir des formulations solubles à pH physiologique par réacétylation du chitosan fongique, mais on constate :

- une dégradation très rapide *in vivo* ou conditions similaires,
- une réaction immunitaire du sujet chez lequel est injecté ou implanté ce chitosane, par exemple par injection ou implantation intra-articulaire, apparaît,
- le chitosane n'est pas adéquat pour les applications visées, et ne permet donc pas une utilisation thérapeutique suffisamment satisfaisante du chitosane, en particulier par injection ou implantation intra-articulaire.

Il existe différentes publications concernant la carboxyalkylation du chitosane, et en particulier la carboxyméthylation du chitosane, essentiellement dans le but de solubiliser le chitosane. En théorie, un chitosane présente une formule sans unité N-acétyl-glucosamine, mais en pratique, le chitosane étant issu de la chitine, qui elle comprend des unités N-acétyl-glucosamine, et le chitosane présente un certain degré d'acétylation (DA), qui est la proportion d'unités N-acétyl-glucosamine du chitosane. Le DA du chitosane est en général faible. Au-delà, surtout au-delà de 30%, il s'agit en général de chitosane réacétylé.

Par ailleurs, la demande de brevet chinois CN1431229A concerne des dérivés de chitosane carboxyméthylé pour leur capacité hydratante, mais se limite à cette caractéristique.

L'art antérieur a également envisagé la carboxyméthylation de la chitine d'origine animale, en particulier crustacée. Mais la chitine d'origine crustacée est difficile à substituer, notamment il faut la congeler et l'alcaliniser pour pouvoir la substituer (voir par exemple la demande de brevet chinois CN106474569). Le procédé est difficile à mettre en œuvre et en particulier à industrialiser. D'autre part, un tel procédé de substitution à partir de chitine d'origine crustacée est coûteux en énergie, peu reproductible, et risque de dégrader le polymère et d'hydrolyser les groupes acétyle des unités N-acétyl-glucosamine de manière significative et difficilement contrôlable.

**Buts de l'invention**

L'invention a pour but de résoudre le problème technique consistant à fournir un dérivé de chitosane adéquat pour être utilisé chez un être humain ou animal, en particulier dans le domaine thérapeutique, rhumatologique, ophtalmologique, médecine esthétique, chirurgie plastique, chirurgie interne, dermatologique, ou cosmétique.

Plus particulièrement, l'invention a pour but de résoudre le problème technique consistant à fournir un dérivé de chitosane adéquat pour être utilisé chez un être humain ou animal dans le domaine thérapeutique, en particulier utilisable à titre de viscosupplément, et en particulier de pouvoir être injectée dans ou en mélange avec un fluide synovial.

L'invention a notamment pour but de résoudre le problème technique consistant à fournir un fluide synovial reconstruit, c'est-à-dire une composition restaurant les propriétés d'une articulation, comme par exemple en lui apportant une capacité à lubrifier les surfaces de cartilage.

L'invention a encore pour but de résoudre le problème technique consistant à fournir un dérivé de chitosane, ou une composition en comprenant, présentant de bonnes propriétés et compatibilité en mélange avec un fluide synovial, et en particulier un fluide synovial d'un être humain ou animal, par exemple pour traiter une pathologie articulaire ou dégénérescence du fluide synovial en cause.

L'invention a encore pour but de résoudre le problème technique consistant à fournir un dérivé de chitosane, ou une composition en comprenant, limitant la réaction immunitaire d'un sujet, et en particulier d'un être humain ou animal, recevant une administration, par exemple par injection, d'un dérivé de chitosane ou une composition en comprenant.

L'invention a encore pour but de résoudre le problème technique consistant à fournir un dérivé de chitosane, ou une composition en comprenant, présentant des caractéristiques peu variables selon le pH.

L'invention a encore pour but de résoudre le problème technique consistant à fournir un dérivé de chitosane, ou une composition en comprenant, présentant une osmolalité et un pH adapté à son utilisation en contact avec un tissu d'un être humain ou animal, et acceptable en termes de durée de vie *in situ,* réaction immunologique et/ou réaction à corps étranger et propriétés biomécaniques, selon l'indication thérapeutique visée, en particulier dans le contexte de la médecine régénérative.

**Description de l'invention**

Il a été découvert de manière surprenante qu'un dérivé de chitosane selon la présente invention permet de résoudre au moins l'un, et de préférence l'ensemble, des problèmes techniques décrits ci-dessus.

Il a été en particulier découvert qu'un dérivé de chitosane d'origine fongique permettait de résoudre au moins l'un, et de préférence l'ensemble, des problèmes techniques décrits ou suggérés. Notamment, un dérivé de chitosane d'origine fongique permet de limiter la réponse immunitaire d'un sujet à qui on a administré, typiquement par injection ou implantation, le dérivé de chitosane ou une composition en comprenant.

La présente invention concerne selon un premier aspect un carboxyalkyl chitosane d'origine fongique présentant des unités glucosamine, des unités N-acétyl- glucosamine et des unités glucosamine substituées par un groupe carboxyalkyl, ledit carboxyalkyl chitosane présentant un degré de substitution par un groupe carboxyalkyl supérieur à 50%, exprimé en nombre de mole du substituant par rapport au nombre de mole d'unités totales.

On parle également de dérivé de chitosane ou de chitosane substitué.

Il a été en particulier découvert également qu'un dérivé de chitosane présentant une charge électrostatique (caractérisée par son potentiel zeta) sur une plage de pH autour du pH du milieu où il est administré, et notamment à pH égal à 7,5, inférieure à une certaine valeur permettait de résoudre au moins l'un, et de préférence l'ensemble, des problèmes techniques décrits ou suggérés. Notamment, un tel dérivé de chitosane permet de limiter la réponse immunitaire d'un sujet à qui on a administré, typiquement par injection ou implantation, le dérivé de chitosane ou une composition en comprenant.

La présente invention concerne selon un deuxième aspect un dérivé de chitosane présentant des unités glucosamine, des unités N-acétyl-glucosamine et des unités glucosamine substituées par un groupe carboxyalkyl, ledit carboxyalkyl chitosane présentant un potentiel zeta, mesuré à pH 7,5, inférieur ou égal à -10 mV, et de préférence inférieur ou égal à -15 mV.

Le chitosane est par exemple référencé sous le numéro CAS 9012-76-4.

Le chitosane utilisé pour l'invention est avantageusement d'origine fongique, et de préférence issu du mycélium d'un champignon du type *Ascomycète,* et en particulier *d'Aspergillus niger,* et/ou d'un champignon *Basidiomycète,* et en particulier *Lentinula edodes* (shiitake) et/ou *Agaricus bisporus* (champignon de Paris). De préférence, le chitosane est issu d'*Agaricus bisporus.* Le chitosane est de préférence très pur, c'est-à-dire contenant peu d'impuretés issues de son origine fongique ou du procédé de fabrication, et d'une qualité microbiologique compatible avec son utilisation comme implant ou composition pharmaceutique. Une méthode de préparation du chitosane est celle décrite dans les brevets WO 03/068824 (EP 1483299 ; US 7 556 946).

En général, la chitine est mise en suspension aqueuse en présence d'hydroxyde de sodium, puis le milieu est porté à haute température pendant une durée variable selon la masse moléculaire désirée. Le chitosane est ensuite purifié par solubilisation en milieu acide et précipité en milieu alcalin, lavé et séché.

De préférence, le chitosane est de grade suffisamment pur pour une utilisation pharmaceutique.

Le chitosane est avantageusement purifié et ensuite de préférence séché. Après purification, le procédé de l'invention peut comprendre une étape de séchage du carboxyalkyl chitosane, puis éventuellement de broyage de celui-ci pour obtenir une poudre. On peut sécher le carboxyalkyl chitosane par exemple par évaporation de l'eau, par exemple par un procédé de spray-drying (atomisation), de lit fluidisé, ou par séchage à la chaleur sous vide ou à pression atmosphérique, ou encore par lyophilisation.

Le carboxyalkyl chitosane peut être solubilisé dans une solution aqueuse, et par exemple dans une eau de qualité pharmaceutique acceptable pour une injection ou implantation dans un corps, et en particulier un corps humain.

Le chitosane préparé peut-être de différentes masses moléculaires, et généralement allant de 10 000 à 500 000.

Selon une variante, la masse moléculaire moyenne est comprise entre 20 000 et 60 000.

Selon une autre variante, la masse moléculaire moyenne est comprise entre 60 000 et 100 000.

Selon une autre variante, la masse moléculaire moyenne comprise entre 100 000 et 120 000.

Selon une autre variante, la masse moléculaire moyenne comprise entre 120 000 et 150 000.

Selon une autre variante, la masse moléculaire moyenne est comprise entre 150 000 et 220 000.

Selon une autre variante, la masse moléculaire moyenne est comprise entre 220 000 et 300 000.

Selon une autre variante, la masse moléculaire moyenne est comprise entre 300 000 et 500 000.

Lorsque le chitosane est réticulé, la masse moléculaire du polymère réticulé peut être bien plus élevée.

Il est possible d'hydrolyser le chitosane afin de diminuer sa masse moléculaire.

De préférence ici, la masse moléculaire moyenne est la masse moléculaire moyenne en viscosité (Mv), calculée à partir de la viscosité intrinsèque selon l'équation de Mark-Houwink. La viscosité intrinsèque est mesurée par viscosimétrie capillaire, avec un viscosimètre capillaire de type Ubbelohde, selon la méthode de la monographie 2.2.9 de la Pharmacopée Européenne. On mesure le temps d'écoulement de la solution à travers un tube capillaire adapté (Lauda, par exemple le tube capillaire Ubbelohde 510 01 de diamètre 0,53mm) à l'aide d'un viscosimètre automatique I-Visc (Lauda), d'abord à la concentration initiale en chitosane, puis pour plusieurs dilutions, par exemple selon les recommandations de la monographie 2.2.9. On en déduit la viscosité intrinsèque réduite pour chacune des concentrations. On porte la viscosité réduite en fonction de la température, et on extrapole la valeur à la concentration 0 pour en déduire la viscosité intrinsèque. Il faut par exemple porter la viscosité réduite ($h_{réd}$ en mL/g) des i dilutions en fonction de la concentration C des i dilutions (g/mL) selon la formule 5.

$$\text{Formule 2.} \quad [h_{réd}] = (t_1 - t_0) - (1 - C).$$

Pour calculer la masse viscosimétrique moyenne, on applique l'équation de Mark-Houwink avec les constantes k et alpha recommandées par Rinaudo et al. (in : Int J Biol Macromol, 15, 281, 1993), selon le degré d'acétylation (DA) du chitosane, selon l'une des trois formules suivantes.

$$\text{Formule 3.} \quad Mv = ([h]/0{,}082)^{(1/0,76)},$$

pour un DA de 2% ;

$$\text{Formule 4.} \quad Mv = ([h]/0{,}076)^{(1/0,76)},$$

pour un DA de 10% (par exemple 11.5%) ;

$$\text{Formule 5.} \quad Mv = ([h]/0{,}074)^{(1/0,76)},$$

pour un DA de 20% (par exemple 21%).

Pour les valeurs de DA intermédiaires, on réalise une interpolation linéaire pour calculer la masse viscosimétrique moyenne (Mv).

De préférence, le chitosane utilisé est d'une masse moléculaire moyenne comprise entre 120 000 et 150 000 ou encore comprise entre 150 000 et 220 000 ou encore comprise entre 220 000 et 300 000, ou encore au-dessus de 300 000, et en général jusqu'à 500 000.

On peut aussi mesurer de la masse moléculaire finale du carboxyalkyl chitosane : on peut par exemple mesurer sa viscosité intrinsèque par viscosité capillaire, en déduire sa masse moléculaire moyenne (Mw) (en déterminant au préalable les paramètres K et alpha du carboxyalkyl chitosane), ou par une méthode par chromatographie par exemple par perméation de gel.

Typiquement, dans le carboxyalkyl chitosane selon l'invention les unités glucosamine sont des unités D-glucosamine (unités D-glucosamine, unités N-acétyl-D-glucosamine, et au moins l'une des unités D-glucosamine et des unités N-acétyl-D-glucosamine étant substituées).

Selon une variante, un chitosane substitué présente une substitution des unités D-glucosamine uniquement.

Selon une autre variante, un chitosane substitué présente une substitution des unités D-glucosamine et N-acétyl-D-glucosamine simultanément, et dans lequel le groupe carboxyalkyl est lié de manière covalente, selon une variante aux groupes amine du chitosane uniquement, ou selon une autre variante aux groupes amine et hydroxyle du chitosane simultanément.

La substitution est en général seulement partielle, toutes les unités ne sont pas nécessairement substituées.

Selon un mode de réalisation, le degré de substitution des unités D-glucosamine exprimé en nombre de moles d'unités D-glucosamine par rapport au nombre de moles d'unités totales (unités D-glucosamine et N-acétyl-D-glucosamine, substituées ou non) du chitosane substitué, va de 30% à 250%.

Le degré de substitution par un groupe carboxyalkyl supérieur à 50%, exprimé en nombre de mole du substituant par rapport au nombre de mole d'unités totales.

Selon un mode de réalisation, le degré de substitution des unités D-glucosamine exprimé en nombre de moles d'unités D-glucosamine par rapport au nombre de moles d'unités totales (unités D-glucosamine et N-acétyl-D-glucosamine, substituées ou non) du chitosane substitué, va de 50% à 200%, et encore de préférence supérieur à 70%.

Selon un mode de réalisation, le degré de substitution par un groupe carboxyalkyl inférieur à 80%, exprimé en nombre de mole du substituant par rapport au nombre de mole d'unités totales.

Typiquement, la substitution se réalise par liaison covalente.

Selon une variante, le carboxyalkyl chitosane est un N,O-carboxyalkyl chitosane. La proportion d'unités substituées par un groupe carboxyalkyl en position O (soit O3 soit O6 des unités glucosamine et/ou N-acétyl-glucosamine) et/ou en position N (des unités glucosamines) varie. Le degré de substitution peut donc être supérieur à 100%.

Avantageusement, le degré de substitution (DS) et le degré d'acétylation (DA) du carboxyalkyl chitosane sont mesurés par RMN du carbone 13 en phase solide, à l'aide d'un Spectromètre Bruker (Avance III HD 400MHz), équipé d'une sonde PH MAS VTN 400SB BL4 N-P/H. Par exemple, le spectre est enregistré à température ambiante, un temps de relaxation compris entre 1 et 8 secondes, un nombre de scans compris entre 64 et 512. Les aires des signaux des carbones sont déterminées après déconvolution. Les carbones considérés sont les suivants : « CH3 acétyle » (carbone du méthyl du groupe acétyle des unités N-acétyl-glucosamine, substituées ou non), « C1 » (carbone en position 1 des unités glucosamine et N-acétyl-glucosamine) et « C=O » (carbone du carbonyl du substituent carboxyméthyl et carbone du carbonyl C=O du groupe acétyle des unités N-acétyl-glucosamine, substituées ou non). Pour déterminer le DS d'un carboxyalkyl chitosan donné, il faut également enregistrer le spectre RMN du carbone 13 du chitosane précurseur de ce carboxyalkyl chitosan. A partir du spectre du chitosan précurseur, on calcule le « ratio CSU », c'est-à-dire le rapport entre l'aire du signal du groupe « CH3 acétyle » (carbone du méthyl du groupe acétyle des unités N-acétyl-glucosamine) et l'aire du signal du « C=0 » (carbone carbonyle du groupe acétyle des unités N-acétyl-D-glucosamine). Le DA du carboxyalkyle chitosane est calculé selon la Formule 1, et le DS selon la Formule 2, où I représente l'aire du signal du carbone considéré.

Formule 1 :

$$DA = \frac{I_{CH3\,ac\acute{e}tyle}}{I_{C_1}}$$

Formule 2 :

$$DS = \frac{I_{C=0} - {}^{I_{CH3}}\!/_{Ratio\,CsU}}{I_{C_1}}$$

On peut déterminer le DA et le DS à l'aide d'autres méthodes connues pour les carboxyalkyl chitosanes, par exemple par RMN du proton en milieu aqueux, à l'aide d'un spectromètre de résonance magnétique, par exemple selon la méthode décrite par Liu et al. (in : Carb Polym 137, 600, 2016), par exemple avec une hydrolyse préalable du carboxyalkyl chitosane y ajoutant une solution concentrée d'acide chlorhydrique deutéré avant analyse.

Si une autre méthode RMN est plus avantageuse pour estimer le degré de substitution de manière fiable, il convient d'utiliser une telle méthode. Les méthodes ci-dessus doivent être adaptée par l'homme du métier en ce qui concerne la préparation de l'échantillon et les signaux à intégrer, notamment en fonction de la résolution, de la robustesse et de la position des protons des signaux à utiliser pour le calcul du degré de substitution.

Le degré de carboxyalkylation du chitosane peut varier avantageusement de 50 à 200%, et par exemple de 70 à 170%, exprimé en nombre de moles de carboxyalkyle par rapport au nombre de moles d'unités totales.

Selon une variante, le degré de carboxyalkylation du chitosane peut varier avantageusement de 70 à 130%, exprimé en nombre de moles de carboxyalkyle par rapport au nombre de moles d'unités totales.

Le degré de substitution du chitosane est typiquement corrélé au ratio massique des réactifs par rapport au chitosane au départ de la réaction. Comme agents carboxyalkylants, on peut citer les chlorures d'acide (ou leurs sels, par exemple le monochloroacétate de sodium), comme par exemple ceux portant un ou plusieurs groupe carboxyméthyle, carboxyéthyle, carboxypropyle, carboxybutyle, etc.

Selon une variante, la présente invention concerne un carboxyalkyle chitosane où la partie alkyl du carboxyalkyl est en C1-C5, linéaire ou ramifiée.

Selon une variante, la présente invention concerne un carboxyméthyle chitosane.

Selon cette variante, le chitosane substitué est un chitosane N-carboxyalkylé.

Selon cette variante, le chitosane substitué est un chitosane O-carboxyalkylé.

Selon cette variante, le chitosane substitué est un chitosane N-carboxyalkylé et O-carboxyalkylé.

Avantageusement, le potentiel zeta, mesuré à pH 7,5, est inférieur ou égal à -18 mV.

Avantageusement, le carboxyalkyl chitosane présente un potentiel zeta, mesuré à pH 7,5, inférieur ou égal à -22 mV, et de préférence inférieur ou égal à -24 mV.

Selon une variante spécifique, le chitosane substitué présente, de préférence une masse moléculaire moyenne de 150 000 à 220 000 et un degré de substitution allant de 50 à 200%, la masse moléculaire étant de préférence exprimée avant substitution.

Selon une autre variante spécifique, le chitosane substituée présente, une masse moléculaire moyenne de 120 000 à 150 000 et un degré de substitution allant de 70 à 200%, la masse moléculaire étant de préférence exprimée avant substitution.

Selon une variante spécifique, le chitosane substitué présente, de préférence une masse moléculaire moyenne de 220 000 à 300 000 et un degré de substitution allant de 70 à 200%, la masse moléculaire étant de préférence exprimée avant substitution.

Selon une autre variante spécifique, le chitosane substituée présente, une masse moléculaire moyenne de 220 000 à 300 000 et un degré de substitution allant de 50 à 200%, la masse moléculaire étant de préférence exprimée avant substitution.

Selon une autre variante spécifique, le chitosane substituée présente, une masse moléculaire moyenne de 300 000 à 500 000 et un degré de substitution allant de 50 à 200%, la masse moléculaire étant de préférence exprimée avant substitution.

Selon une autre variante spécifique, le chitosane substituée présente, une masse moléculaire moyenne de 300 000 à 500 000 et un degré de substitution allant de 70 à 200%, la masse moléculaire étant de préférence exprimée avant substitution.

Selon une variante spécifique, le chitosane substitué présente un degré de substitution allant de 50 à 200%, et de préférence de 70 à 200%, et un degré d'acétylation de 20 à 80%, et de préférence de 30 à 75%.

Selon une variante spécifique, le chitosane substitué présente un degré de substitution allant de 50 à 200%, et de préférence de 70 à 200%, et un degré d'acétylation de 20 à 50%, et de préférence de 20 à 40%.

Selon une variante spécifique, le chitosane substitué présente un degré de substitution allant de 50 à 200%, et de préférence de 70 à 200%, et un degré d'acétylation de 50 à 75%.

Selon une autre variante spécifique, le chitosane substituée présente un degré de substitution allant de 90 à 200%, et de préférence de 90 à 150%, et un degré d'acétylation de 20 à 80%, la masse moléculaire étant de préférence exprimée avant substitution.

Selon une variante spécifique, le chitosane substitué présente un degré de substitution allant de 90 à 200%, et de préférence de 90 à 150%, et un degré d'acétylation de 20 à 50%, et de préférence de 20 à 40%.

Selon une variante spécifique, le chitosane substitué présente un degré de substitution allant de 90 à 200%, et de préférence de 90 à 150%, et un degré d'acétylation de 50 à 75%.

Selon une variante spécifique, le chitosane substitué présente, de préférence une masse moléculaire moyenne de 220 000 à 300 000, un degré de substitution allant de 90 à 200%, et de préférence de 90 à 150%, et un degré d'acétylation de 50 à 75%, la masse moléculaire étant de préférence exprimée avant substitution.

Selon une variante, le carboxyalkyl chitosane est réticulé. Ainsi plusieurs chaines de chitosane pleuvent être réticulées, par exemple par réaction avec un agent réticulant, comme par exemple des agents réticulant utilisés pour la réticulation des polysaccharides, comme par exemple génipine, butyl biglycidyl ether, glutaraldéhyde, epichlorohydrin, 1-bromo-3,4-epoxybutane, 1-bromo-4,5-epoxypentane, l-chloro-2,3-epithio-propane, 1-bromo-2,3-epithiopropane, l-bromo-3,4-epithio-butane, 1-bromo-4,5-epithiopentane, 2,3-dibromopropanol, 2,4-dibromobutanol, 2,5-dibromopentanol, 2,3-dibromopro-panethiol,2,4-dibromobutanethiol, and 2,5-dibromopentane- thiol epichlorohydrin, 2,3-dibromopropanol,1-chloro-2,3-epithiopropane, dimethylaminopropylcarbodiimide, dextran oxydé, acide gallique, gallate d'épigallocatéchine, curcumin, acide tannique, ou encore des composés diisocyanate tel que diisocyanate d'hexaméthylène ou diisocyanate de toluène.

Avec un carboxyalkyl chitosane réticulé la masse moléculaire peut être très élevée.

En substituant le chitosane, il a été possible de préparer une solution d'un carboxyalkyle chitosane soluble dans une solution aqueuse dont le pH varie dans une large gamme, alors que le chitosane non substitué n'est soluble qu'à pH en dessous de 5,5 à 6,5. Le carboxyalkyle chitosane présente ainsi une capacité à être solubilisé à différents pH grâce à la présence de groupes carboxyalkyle qui modifient son profil de solubilité, et en particulier au pH physiologique ou au pH des fluides physiologiques modifiés par une pathologie, par exemple une pathologie inflammatoire. Il est connu que le pH des fluides physiologiques comme le liquide synovial d'une articulation, l'humeur aqueuse, l'humeur vitrée, les larmes, peuvent varier de manière significative entre individus, en raison de différents facteurs comme l'âge, la pathologie, etc. Il est donc avantageux que le carboxyalkyl chitosane puisse rester soluble dans une large gamme de pH, par exemple de 6,0 à 8,5, ou encore de 5,0 à 8,5, ou encore de 4,5 à 8,5.

Selon un mode de réalisation, la formulation de chitosane substitué présente une osmolalité de 100 à 700 mosm/kg, de préférence de 200 à 500 mosm/kg.

Avantageusement, l'osmolalité de la formulation de chitosane substitué est comprise entre 250 et 400 mosm/kg, et de préférence de 275 à 325 mosm/kg.

Selon une variante, la formulation de chitosane substitué présente une osmolalité compatible avec une articulation.

Selon une variante, la formulation de chitosane substitué présente une osmolalité compatible avec une surface oculaire ou intraoculaire.

Il est préférable que l'osmolalité de la formulation de chitosane substitué soit comprise entre 250 et 400, et plus spécifiquement entre 250 et 380 mosm/kg.

Par « soluble dans l'eau », on entend que le carboxyalkyle chitosane ne présente pas de trouble visible à l'œil nu lorsqu'il est mis en solution aqueuse. Plus spécifiquement, on peut confirmer la solubilité, c'est-à-dire l'absence de trouble, d'une solution de carboxyalkyl chitosane à une concentration par exemple de 1% (m/m) dans l'eau ou un tampon, par exemple un tampon phosphate, par une densité optique inférieure à 0,5, et de préférence inférieure à 0,2, mesurée par spectrométrie UV-visible à la longueur d'onde de 500nm en référence à une cuve de référence ne comprenant que le solvant aqueux utilisé pour l'échantillon mesuré, mais en l'absence du chitosane substitué. Une autre méthode consiste en une inspection visuelle selon la monographie 2.9.20 de la Pharmacopée Européenne. Lorsque le chitosane n'est pas suffisamment substitué, la composition n'est pas soluble dans une gamme de pH satisfaisante, par exemple allant de pH 6,0 à pH 8,5, à température ambiante.

Le degré d'acétylation (DA) du chitosane est déterminé comme par exemple décrit dans les demandes de brevet WO 2017009335 et WO 2017009346 par titrage potentiométrique. Le DA peut alternativement être mesuré par d'autres méthodes connues pour le chitosane, comme la RMN du proton, la RMN du carbone 13 en phase solide, la spectrométrie infra-rouge.

Avantageusement, le carboxyalkyl chitosane présente un degré d'acétylation compris entre 5 et 80%, exprimé en nombre de mole unités N-acétyl- glucosamine par rapport au nombre de mole d'unités totales. Le degré d'acétylation est exprimé en nombre d'unités N-acétyl-D-glucosamine par rapport au nombre d'unités totales N-acétyl-D-glucosamine et D-glucosamine présentes.

Avantageusement, le carboxyalkyl chitosane présente un degré d'acétylation compris entre 40 et 80%, exprimé en nombre de mole unités N-acétyl-glucosamine par rapport au nombre de mole d'unités totales.

Selon une variante, le degré d'acétylation va de 5 à 20%.

Selon une variante, le degré d'acétylation va de 15 à 25%.

Selon une variante, le degré d'acétylation va de 20 et 45%.

Selon une variante, le degré d'acétylation va de 20 à 30%.

Selon une variante, le degré d'acétylation va de 25 à 40%.

Selon une variante, le degré d'acétylation va de 40 à 50%.

Selon une variante, le degré d'acétylation va de 50 à 60%.

Selon une variante, le degré d'acétylation va de 60 à 75%.

Le degré d'acétylation est déterminé par RMN du carbone 13 ou par RMN du proton, selon la même méthode que pour la détermination du DS. Le carboxyalkyl chitosane présente avantageusement un degré d'acétylation contrôlé. Par les termes « chitosane ayant un degré d'acétylation contrôlé » on entend un produit dont le degré d'acétylation, c'est-à-dire la proportion des unités N-acétyl-glucosamine, peut être ajustée de manière contrôlée, notamment par une réaction d'acétylation.

Selon une variante, une composition selon l'invention peut se présenter sous la forme d'une solution et peut ne pas être gélifiée par variation de la température (non-thermogélifiable).

Selon une variante, les caractéristiques rhéologiques d'une solution selon l'invention peuvent évoluer avec la température, mais sans passer par une transition sol-gel. Les caractéristiques rhéologiques d'une solution selon l'invention peuvent notamment se constater par le module d'élasticité (G') et/ou le module de perte (G"), ou encore le module complexe G*.

Selon une variante, les caractéristiques rhéologiques d'une solution selon l'invention sont sensiblement constantes quelles que soit la température.

Selon une variante, une composition selon l'invention peut se présenter sous la forme d'une solution et être thermogélifiable.

Selon une variante, une composition selon l'invention peut être se présenter sous la forme d'un gel et ne pas être thermogélifiable.

L'invention permet donc selon une variante, de préparer une composition thermogélifiable fluide à une température inférieure à celle d'utilisation, typiquement à une température inférieure à la température physiologique, par exemple de 37°C, mais qui soit sous forme de gel à la température d'utilisation, typiquement à la température physiologique, par exemple de 37°C, à pH neutre (pH 7) ou à pH physiologique, et par exemple de 7 à 8,5, avec une osmolalité convenable pour l'utilisation envisagée. Il s'agit par exemple d'une osmolalité physiologique.

Selon une variante, la composition thermogélifiable présente une transition sol-gel thermoréversible.

Avantageusement, la présente invention permet de fournir une composition à faible concentration de chitosane substitué.

Avantageusement, la concentration de carboxyalkyl chitosane est inférieure à 10%, par exemple inférieure ou égal à 5%, en masse par rapport à la masse totale de la composition (m/m).

Selon une variante, la concentration de chitosane est inférieure à 4%, par exemple inférieure ou égal à 3%, ou encore par exemple inférieure ou égal à 2% en masse par rapport à la masse totale de la composition (m/m).

Avantageusement, la composition de l'invention peut comprendre également un autre biopolymère que le chitosane substitué. Selon une variante avantageuse, le biopolymère est un polysaccharide, oxydé ou non, réticulé par des liaisons covalentes ou non, par exemple l'acide hyaluronique ou le hyaluronate de sodium.

L'acide hyaluronique peut présenter une masse moléculaire jusqu'à 5 millions Da. La masse moléculaire de l'acide hyaluronique peut être reflétée par sa viscosité intrinsèque ou encore par sa viscosité dynamique en solution. L'acide hyaluronique peut présenter une densité allant de 1 à 4 m3/kg, et par exemple être caractérisé comme de faible (par exemple environ 1 à 2 $m^3$/kg) ou haute (par exemple environ 2 à 4 m3/kg) masse moléculaire.

Avantageusement, la concentration d'acide hyaluronique est inférieure à 4%, par exemple inférieure ou égale à 3%, ou encore par exemple inférieure ou égale à 2% en masse par rapport à la masse totale de la composition (m/m).

Selon une variante spécifique, la concentration d'acide hyaluronique est inférieure à 1,9% (m/m), exprimée en masse par rapport la masse de la composition finale. Avantageusement, la concentration d'acide hyaluronique est comprise entre 0,5 et 1,5% (m/m), exprimée en masse par rapport à la masse de la composition finale. Selon une variante particulière, la concentration d'acide hyaluronique est environ de 0,9%, de 1,0%, de 1,1%, de 1,2%, de 1,3%, de 1,5% (m/m), exprimée en masse par rapport à la masse de la composition finale.

Le ratio entre le chitosane et l'acide hyaluronique peut par exemple varier de 5 à 95 %, par exemple de 10 à 90%, et encore par exemple de 30 à 70% de chitosane substitué et de 5 à 95 %, par exemple de 10 à 90%, et encore par exemple de 30 à 70% respectivement, d'acide hyaluronique, les pourcentages exprimés par rapport : masse sèche de chitosane substitué /masse sèche d'acide hyaluronique). Selon une variante, ce ratio entre le chitosane et l'acide hyaluronique est de 1/1 (soit 50% de chitosane et 50% d'acide hyaluronique). Selon une autre variante, le ratio entre le chitosane et l'acide hyaluronique est de 1,5/0,5 (soit 75% de chitosane et 25% d'acide hyaluronique).

Selon une variante, l'acide hyaluronique peut être réticulé entre différentes chaines d'acide hyaluronique.

La présente invention concerne également un procédé de préparation du carboxyalkyl chitosane.

Selon une variante, le procédé de préparation du carboxyalkyl chitosane selon l'invention comprend la préparation d'un chitosane d'origine fongique, la réacétylation du chitosane et la carboxyalkylation du chitosane réacétylé. Ainsi, l'invention concerne un chitosane réacétylé ou un carboxyalkyl chitosane réacétylé.

Selon un mode de réalisation, on peut ainsi dissoudre du chitosane dans un milieu aqueux, de préférence légèrement acidifié (pH 6 par exemple). On peut ajouter de l'anhydride acétique à la solution de chitosane en une ou plusieurs fois. On ajoute ensuite un agent basique comme par exemple de la soude et/ou de l'urée. On ajoute ensuite un agent alkylant comme par exemple du monochloroacétate de sodium (c'est-à-dire le sel de sodium de l'acide chloroacétique) ou l'acide chloroacétique. Ensuite le chitosane substitué est purifié, récupéré et séché.

Selon une variante, le procédé de préparation du carboxyalkyl chitosane selon l'invention comprend la préparation d'un chitosane, la carboxyalkylation du chitosane, puis la réacétylation du chitosane carboxyalkylé. Avantageusement, une telle méthode permet un contrôle précis du degré d'acétylation du carboxyalkyl chitosane final, et en particulier d'obtenir un degré d'acétylation élevé, par exemple au-dessus de 40%. Ainsi, l'invention concerne un chitosane réacétylé puis carboxyalkylé ou un carboxyalkyl chitosane réacétylé.

Selon une variante, le procédé de préparation du carboxyalkyl chitosane selon l'invention comprend la préparation d'une chitine d'origine fongique, la carboxyalkylation de la chitine, et éventuellement la réacétylation de la chitine carboxyalkylé pour obtenir le carboxyalkyl chitosane selon l'invention.

Selon une variante, le procédé de préparation du chitosane carboxyalkylé selon l'invention comprend la préparation d'une chitine d'origine fongique, une déacétylation de la chitine, la carboxyalkylation de la chitine, et éventuellement la réacétylation de la chitine carboxyalkylée pour obtenir le carboxyalkyl chitosane selon l'invention.

Selon un mode de réalisation, l'étape de carboxyalkylation comprend une étape d'alcalinisation des groupes hydroxyles du chitosane, pour favoriser un degré de substitution élevé, et par exemple à la fois en position N-des unités de glucosamine et Odes unités de glucosamine et N-acetyl-glucosamine.

Selon un mode de réalisation, le procédé de l'invention comprend une étape d'alcalinisation comprenant la dispersion de chitosane dans d'une solution d'alcool, comme par exemple l'isopropanol en présence d'un agent basique comme par exemple la soude, et l'agitation pendant une durée d'au moins une heure à une température de minimum -32°C et de préférence maximum 15°C. A cette suspension on ajoute ensuite un agent alkylant comme par exemple l'acide monochloroacétique. Ensuite le chitosane substitué est purifié, récupéré, puis séché.

Selon une variante, le procédé de préparation du chitosane carboxyalkylé selon l'invention comprend la préparation d'un chitosane, la carboxyalkylation du chitosane puis la réacétylation du chitosane carboxyalkylé.

Selon un mode de réalisation, l'étape de carboxyalkylation ne comprend pas d'étape d'alcalinisation.

L'étape de réacétylation d'un carboxyalkyl chitosane peut par exemple comprendre un ou plusieurs ajouts d'anhydride acétique à la solution de carboxyalkyl chitosane.

Les étapes finales de purification, filtration et séchage pour récolter le carboxyalkyle chitosane purifié sont réalisées selon des méthodes connues en vue d'obtenir un carboxyalkyl chitosane selon le degré de pureté souhaité, qui dépend en général de l'application envisagée, comme par exemple par cycles de précipitation et solubilisation ou par dialyse.

Selon une variante, le ratio anhydride acétique/chitosane (volume/masse) varie entre 0,1 et 10, et de préférence de 0,1 à 2, lors de l'étape de réacétylation.

Selon une variante, le ratio massique agent carboxyalkylant/chitosane varie entre 1 et 50, et de préférence de 2,5 et 25, lors de l'étape de carboxyalkylation.

Selon une variante, on utilise un ratio massique des réactifs apportant au chitosane le groupe carboxyalkyle (c'est-à-dire l'agent alkylant) par rapport à l'agent basique nécessaire et suffisant pour obtenir le degré de substitution souhaité. Par exemple, le ratio massique de l'agent alkylant par rapport à l'agent basique est supérieur à 1 et inférieur à 10, et de préférence va de 1,4 à 3,3.

L'invention concerne également un procédé de préparation d'une composition selon l'invention.

Selon une variante, le procédé comprend typiquement :

- la dissolution d'un chitosane substitué dans une solution aqueuse, de préférence dans une solution tamponnée à pH de préférence compris entre 6,2 et 8,5 et de préférence entre 6,5 et 7,5 ;
- l'ajustement éventuel du pH à un pH désiré, en général au pH physiologique pour l'application visée, par exemple par ajout d'un agent tamponnant, d'un acide ou d'une base ;
- l'ajout éventuel d'autres excipients, comme par exemple un sucre réducteur, par exemple le sorbitol ou le mannitol ;
- l'ajustement éventuel de l'osmolalité finale de la composition.

Avantageusement, le procédé comprend également une étape ultérieure de remplissage d'un dispositif d'injection ou d'implantation, comme par exemple une seringue, avec le carboxyalkyl chitosane ou la composition le comprenant. Avantageusement, le dispositif d'injection, comme par exemple une seringue, peut ensuite subir une stérilisation à la vapeur. Ce dispositif, par exemple une seringue, peut ensuite être emballé, de préférence de manière stérile. Ce peut être également une poche, une flapule, ou un flacon permettant l'instillation de la solution de carboxyalkyl chitosane.

Il est avantageux d'utiliser un chitosane présentant un degré de pureté suffisant pour l'application envisagée.

Avantageusement, le procédé comprend également une étape ultérieure de remplissage d'un dispositif d'injection, d'implantation ou d'instillation, comme par exemple une seringue, avec la composition selon l'invention. Avantageusement, le dispositif d'injection, comme par exemple une seringue peut ensuite subir une stérilisation à la vapeur. Ce dispositif, par exemple une seringue, peut ensuite être emballée, de préférence de manière stérile.

Selon une variante, le carboxyalkyl chitosane selon l'invention est stérilisé par filtration et/ou par stérilisation à la vapeur, avant remplissage d'un dispositif d'injection, d'implantation ou d'instillation, comme par exemple une seringue ou un flacon.

Il est avantageux d'utiliser un chitosane présentant un degré de pureté suffisant pour l'application envisagée.

La présente invention concerne plus particulièrement une composition injectable comprenant le carboxyalkyl chitosane selon l'invention.

L'invention concerne aussi une composition pharmaceutique comprenant au moins un carboxyalkyl chitosane défini selon l'invention.

Selon une variante, le carboxyalkyl chitosane ou la composition en comprenant est utilisé comme composition pharmaceutique injectable, implantable ou apte à l'instillation, ou dispositif médical injectable ou implantable ou apte à l'instillation.

L'invention couvre encore un carboxyalkyl chitosane ou une composition le comprenant, sous une forme sèche, notamment sous une forme lyophilisée. On peut notamment (re)disperser, et de préférence solubiliser, le produit lyophilisé avant usage.

La présente invention concerne plus particulièrement une composition selon l'invention pour une utilisation pour un traitement thérapeutique, par exemple comprenant l'injection par voie sous-cutanée, intradermique, intraoculaire, ou intra-articulaire, de ladite composition, par exemple pour la réparation, la régénération ou le comblement d'au moins un tissu corporel nécessitant une réparation ou un comblement.

Avantageusement, selon un mode de réalisation, un carboxyalkyl chitosane selon l'invention présente une immunocompatibilité des formulations de carboxyméthyl chitosane d'origine fongique à l'aide du modèle de poche à air chez la souris, 24 heures après injection exprimée en nombre de globules blancs inférieure à $10 \times 10^6$ cellules/ mL, et de préférence inférieure à $8 \times 10^6$ cellules/ mL.

Avantageusement, selon un mode de réalisation, un carboxyalkyl chitosane selon l'invention présente une immunocompatibilité des formulations de carboxyméthyl chitosane d'origine fongique à l'aide du modèle de poche à air chez la souris, 24 heures après injection exprimée en concentration de IL1-beta inférieure à $10 \times 10^{-9}$g/mL, et de préférence inférieur à $5 \times 10^{-9}$g/mL.

Avantageusement, selon un mode de réalisation, un carboxyalkyl chitosane selon l'invention présente une immunocompatibilité des formulations de carboxyméthyl chitosane d'origine fongique à l'aide du modèle de poche à air chez la souris, 24 heures après injection exprimée en concentration de KC/CXCL1 inférieure à $50 \times 10^{-9}$g/mL, et de préférence inférieur à $30 \times 10^{-9}$g/mL.

Avantageusement, selon un mode de réalisation, un carboxyalkyl chitosane selon l'invention présente une immunocompatibilité des formulations de carboxyméthyl chitosane d'origine fongique à l'aide du modèle de poche à air chez la souris, 24 heures après injection exprimée en concentration de TNF-alpha inférieure à $150 \times 10^{-9}$g/mL, et de préférence inférieur à $125 \times 10^{-9}$g/mL.

Avantageusement, selon un mode de réalisation, un carboxyalkyl chitosane selon l'invention présente une immunocompatibilité des formulations de carboxyméthyl chitosane d'origine fongique à l'aide du modèle de poche à air chez la souris, 24 heures après injection exprimée en concentration de KC/CXCL1 inférieure à $50 \times 10^{-9}$g/mL, et de préférence inférieur à $30 \times 10^{-9}$g/mL.

Avantageusement, selon un mode de réalisation, un carboxyalkyl chitosane selon l'invention présente une immunocompatibilité des formulations de carboxyméthyl chitosane d'origine fongique à l'aide du modèle de poche à air chez la souris, 24 heures après injection, satisfaisant simultanément les limites maximales exprimés ci-dessus en nombre de globules blanc et des concentrations en IL1-beta, KC/CXCL1 et TNF-alpha.

Les mesures du nombre de globules blanc et des concentrations en IL1-beta, KC/CXCL1 et TNF-alpha sont réalisées selon les protocoles des exemples.

Avantageusement, selon un mode de réalisation, un carboxyalkyl chitosane selon l'invention présente un temps de demi-vie en présence d'un mélange d'enzymes lysozyme et hyaluronidase à 37°C supérieur à 500 minutes, selon le protocole de mesure de l'exemple 6.

Avantageusement, selon un mode de réalisation, en particulier en application intra-articulaire, un carboxyalkyl chitosane selon l'invention présente un coefficient de friction ($COF_0$) inférieur à 5, et de préférence inférieur ou égal à 4 (selon le protocole de l'exemple 9).

La présente invention concerne notamment une composition selon l'invention pour une utilisation pour un traitement en rhumatologique, en ophtalmologique, en médecine esthétique, en chirurgie plastique, en chirurgie interne, par exemple pour la prévention des adhérences tissulaires post-chirurgicales, en dermatologique ou en cosmétique.

Selon une variante, le tissu corporel est choisi parmi les tissus appartenant aux cordes vocales, muscles, ligaments, tendons, cartilages, organes sexuels, os, articulations, yeux, derme, épiderme, une ou plusieurs couches de la peau, le mésoderme, ou l'une quelconque de leurs combinaisons, et plus particulièrement aux yeux, au derme et aux joints articulaires.

La présente invention concerne également une composition selon l'invention pour une utilisation pour un traitement thérapeutique d'un syndrome de l'œil sec, d'une lésion de cornée ou d'une inflammation articulaire.

La présente invention concerne en outre l'application d'une composition selon l'invention par instillation sur la surface oculaire pour prévenir ou lutter contre une lésion de cornée, ou syndrome de l'œil sec, en particulier dans le but de lubrifier ou régénérer la surface oculaire.

Ainsi, l'invention concerne également une composition de gouttes ophtalmiques comprenant un carboxyalkyl chitosane défini selon la présente invention.

Selon une variante, le sujet est affecté par une pathologie inflammatoire (e.g. ostéoarthrose).

La présente invention concerne plus particulièrement une composition selon l'invention pour le traitement d'une arthrose, d'une arthrite, ou la réparation d'un défaut de cartilage, par exemple par injection dans la cavité synoviale ou par implantation au niveau du défaut de cartilage.

La présente invention concerne plus particulièrement un dispositif médical, par exemple implant médical, caractérisé en ce qu'il comprend ou consiste en une composition selon l'invention.

Selon une variante préférée, l'invention concerne donc un dispositif médical comprenant une chambre contenant un carboxyalkyl chitosane sous une forme sèche, notamment sous une forme lyophilisée, et éventuellement une ou plusieurs autres chambres contenant un ou plusieurs produits actifs, additifs ou excipients.

La composition selon la présente invention peut également comprendre un ou plusieurs additifs ou excipients permettant de moduler ses propriétés.

La présente invention concerne également une composition selon l'invention pour une utilisation dans une méthode de traitement thérapeutique, par exemple comprenant l'instillation ou l'injection par voie sous-cutanée, intradermique, oculaire, intraoculaire, ou intra-articulaire, de ladite composition, par exemple pour la réparation ou le comblement d'au moins un tissu corporel nécessitant une réparation ou un comblement.

La présente invention concerne également une composition selon l'invention pour son utilisation dans une méthode de traitement d'une arthrose, ou la réparation d'un défaut de cartilage, par exemple par injection dans le fluide synovial ou après mélange avec le sang et implantation dans le cartilage.

La présente invention concerne également une composition selon l'invention pour une utilisation dans une méthode de traitement ou de soin esthétique par comblement du derme (« dermal filling »). Il s'agit notamment par exemple d'injecter une composition selon l'invention en sous-cutané ou en intradermique.

La présente invention concerne également une composition selon l'invention pour une utilisation dans une méthode de traitement superficiel de la peau par injection multiple par voie intradermique. De telles compositions peuvent être typiquement utilisées en dermatologie, comme traitements à visée esthétique. Une telle méthode a par exemple pour but de regonfler la peau pour lui faire perdre un aspect fripé (traitement des rides et/ou ridules). Un tel traitement peut être adressé à un sujet souhaitant donner un aspect rajeuni à sa peau.

La présente invention concerne également une composition selon l'invention pour une utilisation dans une méthode de traitement dans laquelle la composition est un agent de viscosupplémentation. Il s'agit ici par exemple d'injecter au niveau intra-articulaire la composition de l'invention notamment pour limiter les frottements des surfaces de cartilage de l'articulation.

La présente invention concerne également une composition selon l'invention pour une utilisation comme vecteur cellulaire, d'un ou plusieurs types cellulaires, et/ou un ou plusieurs d'agents actifs. Il peut s'agir d'agents actifs d'un point de vue pharmaceutique ou biologique. La composition de l'invention peut en effet être compatible avec la présence de cellules, de préférence de cellules vivantes. Parmi les cellules vivantes d'intérêt, on peut citer par exemple : chondrocytes (cartilage articulaire), fibrochondrocytes (ménisque), fibroblastes de ligament (ligament), fibroblastes de peau (peau), ténocytes (tendons), myofibroblastes (muscle), les cellules souches mésenchymales, les globules rouges (sang) et kératinocytes (peau). La composition de l'invention peut également être visée comme vecteur thérapeutique pour la délivrance ciblée et/ou à libération contrôlée d'au moins un agent thérapeutique.

Selon une variante, on ajoute du sang, ou du plasma, ou un lysat plaquettaire, ou du plasma riche en plaquettes, ou tout fluide biologique avec la composition de l'invention permettant par exemple d'augmenter les performances du produit.

Selon une variante, la composition selon l'invention est formulée sous une forme solide (par exemple un film ou une mousse poreuse), qui se solubilise une fois implantée.

Selon une variante, la composition est formulée sous une forme d'une composition nébulisable (spray).

La présente invention concerne également une composition selon l'invention pour une utilisation dans une méthode de traitement ou de soin esthétique d'un ou plusieurs tissus ou organes affectés par une température excessive, comme dans le cas d'une brûlure.

La présente invention concerne également une composition selon l'invention pour une utilisation dans une méthode de traitement de réparation du cartilage (par exemple par implantation sur un défaut de cartilage en vue de promouvoir sa régénération.

La présente invention concerne également une composition selon l'invention pour une utilisation dans une méthode de traitement de prévention des adhérences tissulaires après chirurgie : le produit est appliqué sur les tissus en fin de chirurgie, par exemple gynécologique, abdominale, etc.

La présente invention concerne également une composition à titre de fluide synovial artificiel comprenant un carboxyalkyl chitosane selon l'invention.

La composition selon la présente invention permet de mimer un fluide synovial sain ou d'améliorer un fluide synovial sain ou défectueux en cherchant par exemple à améliorer sa capacité lubrifiante pour réduire les frictions dans l'articulation, et/ou ses propriétés d'absorption des chocs (identifiable par le module d'élasticité G'), tout en étant facilement injectable pour remplir une seringue par exemple ou être injectée dans le corps humain ou animal. Pour indication, le module élastique G' du liquide synovial sain est compris entre 40 et 100Pa, et son module de perte G" est compris entre 1 et 10Pa.

Selon une variante, le carboxyalkyl chitosane, ou la composition en comprenant, est injecté sous forme de solution. Avantageusement, selon cette variante, le carboxyalkyl chitosane, ou la composition en comprenant, est facilement injectable au travers d'une aiguille fine, par exemple une aiguille de diamètre 21 Gauge, à température ambiante. Par injection « facile », on entend de préférence que la force à exercer sur une telle seringue est inférieure à 50 Newton pour faire écouler une composition selon l'invention au travers d'une aiguille de 21 Gauge, de préférence une force inférieure à 20 Newton.

La présente invention concerne également une composition à titre de larmes artificielles comprenant un carboxyalkyl chitosane selon l'invention.

En général, les gammes de valeurs d'osmolalité et de pH recherchées dans les applications biomédicales sont proches des gammes suivantes :

-Osmolalité :

- Iso-osmolaire au plasma : 285 à 295 mosm/kg ;
- Iso-osmolaire au liquide synovial : 404 ± 57 mosml/kg, d'après « Clin Orthop Relat Res, 235, 289-95, 1988 » et « Biochem Biophys Res Comm, 422, 455-461, 2012 » ;
- De 280 à 350 mosm/kg.

-pH :

Un pH physiologique est en général au-dessus de 6,8, en particulier au-dessus de 7,0, et en particulier de 7,4 (comme pour le plasma ou un liquide synovial).

Le pH du plasma est en général de 7,4. Le pH du liquide synovial est en général de 7,768 +/- 0,044 selon « J Bone Joint Surg Br, 41-B(2), 388-400, 1959 » ; ou de 7,3 selon « Acta Orthop Scand 634-641, 1969,», ou encore d'après « Clin Rheumatol 25, 886-888, 2006».

Le pH synovial dans les cas d'ostéoarthrose ou d'arthrite est considéré comme étant en général plus faible que celui du fluide synovial sain.

Ainsi, la présente invention concerne un mélange d'un fluide synovial avec une composition selon la présente invention, par exemple selon un ratio volumique entre (i) le carboxyalkyl chitosane, ou la composition en comprenant, et (ii) le fluide synovial allant de 20/80 à 80/20 (v/v), et par exemple de 50/50 (v/v).

Avantageusement, la composition selon la présente invention est stérile. Très avantageusement, la composition selon la présente invention est stérilisée par montée en température, de préférence sous autoclave.

Selon une variante, les compositions de l'invention sont transparentes ou translucides.

Par « translucide » on entend que l'on peut distinguer un objet lorsqu'on place sa composition entre l'œil de l'observateur et l'objet. Par « transparente » on entend que l'on peut distinguer des caractères alphanumériques lorsqu'on place la composition entre l'œil de l'observateur et les caractères observés. En général on réalise cette évaluation avec une épaisseur de composition d'environ 1cm. On peut également suivre la méthode de la monographie 2.9.20 de la Pharmacopée Européenne pour l'inspection visuelle. On peut également mesurer la densité optique de la composition, par exemple par spectrométrie UV-visible à 500nm et s'assurer que la densité optique est inférieure à 0,5, de préférence 0,2 par rapport à un solvant de référence.

Selon une variante, les compositions de l'invention ne sont pas ou peu opalescentes.

Par « opalescent » on entend que la solution entraîne une diffraction de la lumière visible à l'œil nu, par exemple par inspection visuelle selon une méthode telle que la monographie 2.9.20 de la Pharmacopée Européenne et par comparaison à des solutions de référence de niveaux d'opalescence différents de la Pharmacopée Européenne. Selon une variante, la composition de l'invention est incolore, c'est-à-dire en particulier qu'un observateur à l'œil nu n'attribue pas de couleur spécifique à la composition. Selon une variante, l'opalescence est inférieure au maximum toléré pour l'application envisagée.

L'invention concerne en particulier des articles ou packaging, de préférence stériles, comprenant un ou plusieurs dispositifs d'instillation ou d'injection pré-remplis d'une composition selon l'invention. Il s'agit typiquement de de dispositifs permettant d'instiller le produit sous forme de gouttes ou de seringues pré-remplies

La composition de l'invention peut avantageusement être stérilisée. Ainsi, l'invention concerne un carboxyalkyl chitosane stérilisé. Le carboxyalkyl chitosane est donc ainsi stérile, notamment pour des applications le nécessitant.

Selon une variante, la composition de l'invention est stérilisée à la vapeur, par exemple par une élévation de la température à une température supérieure à 100°C, et de préférence supérieure à 120°C, par exemple entre 121 et 138°C, sous autoclave, pendant une durée suffisante à la stérilisation, par exemple en général de 15 à 20 minutes. Selon une autre variante, la composition peut être stérilisée par filtration à l'aide de filtres prévus à cet effet, par exemple des filtres de porosité inférieure ou égale à 0,2$\mu$m.

Avantageusement, selon un mode de réalisation préféré, la perte en viscosité intrinsèque du carboxyalkyl chitosane lors d'une stérilisation à la vapeur est inférieure à 40%.

L'invention couvre encore une composition de l'invention sous une forme sèche, notamment sous une forme lyophilisée.

On peut notamment (re)disperser cette composition lyophilisée avant usage.

La présente invention couvre également une méthode de traitement thérapeutique comprenant l'injection d'une composition selon l'invention.

La présente invention couvre également l'utilisation d'une composition selon l'invention pour la préparation d'une composition pharmaceutique, en particulier pour un traitement thérapeutique, par exemple comme défini plus spécifiquement par l'invention.

La présente invention couvre également une méthode de soin esthétique, en d'autres termes non-thérapeutique, comprenant l'injection d'une composition selon l'invention. Il s'agit par exemple du comblement de rides ou du comblement d'une ou plusieurs zones de tissu visible endommagées, par exemple suite à un accident ou une intervention chirurgicale, dans un but esthétique.

Un tissu est un ensemble de cellules semblables et de même origine, regroupées en ensemble fonctionnel, c'est-à-dire concourant à une même fonction. Parmi les tissus on peut citer : le tissu épithélial, le tissu conjonctif, le tissu musculaire, et le tissu nerveux.

On entend par « composition selon l'invention » ou des termes équivalents, une composition définie telle que dans la présente invention, y compris selon l'une quelconques des variantes, modes de réalisation particuliers ou spécifiques, indépendamment ou selon l'une quelconque de leurs combinaisons, y compris selon les caractéristiques préférées.

D'autres buts, caractéristiques et avantages de l'invention apparaîtront clairement à l'homme de l'art suite à la lecture de la description explicative qui fait référence à des exemples qui sont donnés seulement à titre d'illustration et qui ne sauraient en aucune façon limiter la portée de l'invention.

Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité.

Ainsi, chaque exemple a une portée générale.

D'autre part, dans les exemples, tous les pourcentages sont donnés en masse, sauf indication contraire, et la température est exprimée en degré Celsius sauf indication contraire, et la pression est la pression atmosphérique, sauf indication contraire.

## Exemples

Les chitosanes précurseurs des chitosanes substitués selon l'invention présentent une masse moléculaire moyenne en viscosité (déterminée par viscosimétrie capillaire) et un degré d'acétylation (DA, proportion d'unité N-acétyl-D-glucosamine, déterminée par titrage potentiométrique) compris dans les gammes du Tableau 1. La masse moléculaire d'un chitosane peut également être définie par la viscosité dynamique d'une solution de concentration 1% (m/m) en chitosane dans l'acide acétique de concentration 1% (v/v), mesurée par viscosimétrie à mobile tournant, par exemple un viscosimètre Brookfield, comme décrit dans le Tableau 1.

| Tableau 1. Caractéristiques des chitosanes précurseurs | | | |
|---|---|---|---|
| Gamme | Gamme de masse moléculaire moyenne (viscosimétrie capillaire) | Gamme de viscosité à 1 % (mPa.s) | Gamme de DA (mol%) |
| « Ultra low » | environ 20 000 - 60 000 | 5-20 | 5 - 20% |
| « Low » | environ 60 000 - 120 000 | 20 - 50 | 15 - 25% |
| « Medium » | environ 120 000 - 150 000 | 50 - 80 | 20 - 30% |
| « High » | environ 150 000 - 220 000 | 80 - 120 | 25 - 40% |
| « Ultra-high » | environ 220 000 to 300 000 | 120 - 200 | 25 - 40% |
| « 400k » | environ 300,000 - 500 000 | 200 - 600 | 35 - 50% |

Les méthodes suivantes sont utilisées dans la présente invention, sauf mention contraire :

Méthode de mesure du potentiel zeta

La formulation à analyser est diluée dans un tampon phosphate pour obtenir une concentration finale en polymère de 0,05%, puis légèrement agitée jusqu'à homogénéisation. La solution est ensuite séparée en différentes fractions, et le pH de chacune des fractions est ajusté à la valeur désirée, entre pH 4 et 8, soit par ajout d'hydroxyde de sodium à 0,1N soit par ajout d'acide chlorhydrique à 0,1 N. Le potentiel zeta de chaque fraction est mesuré à l'aide d'un appareil « Nano-Z » (gamme Zeta-Sizer, Malvern Instruments).

Méthode de mesure de la plage de solubilité des polymères de chitosane

La plage de solubilité est établie en préparant une solution du polymère à tester à une concentration de 1% et un pH de 9, en le fractionnant en plusieurs fractions dont le pH est ajusté à différents pH sur une gamme de 9 à 1. On

14

vérifie pour chaque fraction que le polymère est soluble, c'est-à-dire qu'il ne forme pas de trouble, selon la méthode d'inspection visuelle de la monographie 2.9.20 de la Pharmacopée Européenne. On note la plage de pH sur laquelle le polymère est soluble ou insoluble.

## Modèle de poche à air sous-cutanée chez la souris

Un modèle de poche à air sous-cutanée chez la souris a été utilisé pour évaluer la réaction à corps étranger et l'immunocompatibilité de différents polymères dérivés de chitosan d'origine fongique. Dans ce modèle, une cavité de poche d'air est produite par des injections sous-cutanées répétées d'air stérile dans le dos d'une souris. La poche générée par le gonflement de l'air imite la cavité synoviale, fournissant un environnement localisé dans lequel il est possible d'étudier la stimulation d'une réponse inflammatoire dans le liquide prélevé de la poche d'air et les tissus environnants, tel que décrit par Segwick et al. (in : J Pathol 141, 483, 1983) et Sin et al. (in : Ann Rheum Dis 45, 873, 1986). La cavité de la poche d'air permet d'injecter jusqu'à 1 mL de produit chez un animal d'environ 30 g. Selon le protocole décrit par Dawson et al. (in : Int J Tiss Reac 8, 171, 1991), la poche d'air est établie chez des souris CD-1 Swiss albinos mâles non consanguines (exemptes de pathogènes, poids corporel à la réception 25 à 35 g) aux délais Jour 0 et Jour 4 par l'injection répétée de 5 mL puis 3 mL d'air stérile. Au Jour 7, une injection sous-cutanée unique de 1 mL de produit est effectuée directement dans la cavité de la poche à air. Des injections de 1 mL de salin et d'une solution à 1% de carraghénane servent respectivement de contrôle négatif et positif. Les animaux sont suivis régulièrement dans les heures suivant l'injection pour détecter d'éventuels signes cliniques de toxicité systémique ou locale, et une prise de poids corporel et de température est réalisée au moment de l'injection et du sacrifice. Les animaux sont euthanasiés après une période de contact avec le produit de 24 heures (3 animaux par produit). Les évaluations immunologiques du fluide de lavage récupéré de la poche incluent l'analyse cytologique (comptage et distribution en globules blancs et rouges) et la quantification des principaux médiateurs inflammatoires (IL-1-beta, TNF-alpha et KC/CXCL1) au moyen de kits 'ELISA' (AbCam), réalisées selon les instructions. L'analyse macroscopique et l'histopathologie par coloration hématoxyline/éosine des tissus cutanés environnants est réalisée. Une attention particulière est accordée à la localisation du produit sur les tissus environnants ainsi qu'à sa résorption dans le fluide.

## Modèle d'évaluation de la tolérance locale intra-articulaire dans un modèle ovin

Selon la littérature et les recommandations de l'OARSI, le mouton est reconnu comme un modèle bien caractérisé pour évaluer les effets de l'injection intra-articulaire de traitements innovants (Little et al. in : Osteoarthritis Cartilage 18, S80, 2010 ; Edouard et al. in : Phys Ther Sport 14, 116, 2013 ; McCoy et al. in : Vet Pathol 52, 803, 2015). Le modèle mouton permet d'injecter par voie intra-articulaire un volume de formulation qui simule l'utilisation clinique de la viscosupplémentation chez l'homme (Fraser et al. in : Semin Arthritis Rheum 22, 9, 1993). Les mesures de tolérabilité locale comprennent les signes cliniques d'inflammation synoviale (effusion, boiterie, confort) au moyen d'échelles cliniques semi-quantitatives validées, décrites par Shafford et al. (in : Vet Anaesth Analg 31, 20, 2004) et Kaler et al. (in : Vet J 180, 189, 2009), ainsi que l'évaluation cytologique du fluide synovial. Dans certains cas, ces analyses peuvent être complétées par l'évaluation anatomique et histologique du membre injecté. Un volume de 2mL de formulation est injecté dans l'articulation (grasset) de brebis en bonne santé (âge 2 à 6 ans ; poids 60 à 80 kg). Après injection, les signes cliniques des animaux sont consignés quotidiennement durant une période de 15 jours, selon une échelle semi-quantitative de 0 à 4 pour l'effusion (par palpation du grasset) et la boiterie. On rapporte le nombre de signes rencontrés pour chacun des scores sur toute la durée d'observation. Afin d'évaluer les effets d'une injection répétée d'une même formulation, on réalise une deuxième injection après 1 mois dans l'articulation des mêmes animaux, et on suit les animaux selon le même protocole que pour la première injection. De plus, une ponction du liquide synovial est réalisée au jour 15, et les paramètres macroscopiques (couleur, viscosité), cytologiques (comptage et distribution en globules blancs et rouges), ainsi que la quantité en protéines sériques totales sont déterminés selon des méthodes classiques. Le liquide synovial est considéré comme normal si son aspect est clair, visqueux et légèrement jaunâtre, et que l'analyse cytologique indique que le nombre de globules blancs est inférieur à $1 \times 10^5$ cellules/mL et que la concentration en protéines totales est d'environ 25 mg/mL.

## Exemple 1 - N-succinyl-chitosanes

Des N-succinyl chitosanes de différentes masse moléculaire et structure moléculaire (degré d'acétylation (DA), et degré de substitution par le groupe succinyl (DS)) ont été préparés et caractérisés selon le procédé général décrit dans la demande WO 2016/016463 ou WO 2016/016464 (brevet EP 3016663). Une première étape d'acétylation par addition d'anhydride acétique a été réalisée afin d'augmenter le degré d'acétylation, sauf pour le polymère CSS-1 (Tableau 1a).

Des formulations ont été préparées avec ces N-succinyl chitosanes (CSS) à une concentration de 2% à pH et osmolarité physiologiques, et conditionnées dans une seringue en verre. Les seringues sont stérilisées par autoclave avec un cycle standard (15 minutes à 121°C). On vérifie que les formulations ne présentent pas d'insolubles ou de trouble sur une large gamme de pH autour du pH physiologique (pH 6,5 à 7,5), par inspection visuelle selon la méthode EP 2.9.20. On contrôle que le niveau d'endotoxines bactériennes des formulations est inférieur à 20 EU/mL selon la méthode de la monographie 2.6.14(D) de la Pharmacopée Européenne et que la charge microbiologique est inférieure à 100 cfu/g selon la méthode 2.6.12 de la Pharmacopée Européenne, avant de réaliser une évaluation de leur immunocompatibilité *in vivo*.

| Tableau 1a - Caractéristiques des N-succinyl chitosanes | | | |
|---|---|---|---|
| No | Gamme de masse moléculaire* | DA** (mol %) | DS** (mol %) |
| CSS-1 | Medium | 12 | 62 |
| CSS-6 | Low | 50 | 34 |
| CSS-8 | Low | 49 | 32 |
| CSS-10 | High | 61 | 37 |
| *masse moléculaire du chitosane précurseur, selon Tableau 1 ; **DA et DS mesurés par RMN du proton selon la méthode décrite dans le brevet EP 3016663. | | | |

L'immunocompatibilité des formulations de CSS est évaluée à l'aide du modèle de la poche à air sous-cutanée chez la souris (Tableau 1b). La réaction observée est comparée à celle observée après injection d'une solution de salin (contrôle négatif), d'un contrôle positif très réactif (solution à 1% de carraghénane) et d'un produit commercial à base de Hylan GF-20, un acide hyaluronique partiellement réticulé (Synvisc®, Sanofi).

| Tableau 1b - Immunocompatibilité des formulations de N-succinyl chitosane à l'aide du modèle de poche à air chez la souris, 24 heures après injection* | | | | |
|---|---|---|---|---|
| No | Nbe de globules blancs ($10^6$ cell/ mL) | Conc. en IL1-beta ($10^{-9}$ g/mL)** | Conc. en KC/CXCL1 ($10^{-9}$ g/mL) | Conc.en TNF-alpha ($10^{-9}$ g/mL)** |
| CSS-1 | 1,5 | / | / | / |
| CSS-6 | 0,7 | <3 | 137 | < 125 |
| CSS-8 | 3,7 | / | / | / |
| CSS-10 | 2,1 | < 3 | >700 | < 125 |
| Contrôles | | | | |
| Contrôle négatif (salin) | 0,2 | 17 | 23 | 272 |
| Contrôle positif (1% carraghénane) | 33,2 | 28 | 700 | 767 |
| Synvisc® (Hylan GF-20) | 0,7 | 4 | 46 | < 125 |
| *valeur moyenne sur 3 animaux ; **limite de détection : $3.10^{-9}$ g/mL pour IL1-beta et $125.10^{-9}$ g/mL pour TNF-alpha. | | | | |

D'une manière générale, il ressort que ces formulations à base de CSS entraînent une réaction immunologique de type réaction à corps étranger, mise en évidence par un nombre de globules blancs dans l'infiltrat cellulaire plus élevé que pour le contrôle négatif, néanmoins moins élevé que pour le contrôle positif (1% carraghénane). La réaction induite par les formulations est caractérisée par une activation des neutrophiles, mise en évidence par une concentration en marqueur KC/CXCL1 plus élevée que celles du contrôle négatif et du produit Synvisc®. Les niveaux des marqueurs IL1-beta et TNF-alpha sont quant à eux faibles.

Le fait de modifier le DA et/ou le DS des CSS ou bien de modifier sa masse moléculaire n'entraînent pas de différence de réaction immunologique. Bien que les formulations de CSS ne causent pas d'effet délétère sur les tissus au niveau local, il est nécessaire de pouvoir obtenir un niveau d'immunocompatibilité plus élevé dans le cas d'applications thérapeutiques qui visent des tissus fragilisés par la pathologie concernée, comme par exemple le syndrome de l'œil sec, les lésions de cornée ou les inflammations des pathologies articulaires.

**Exemple 2 - Carboxymethyl chitosanes d'origine animale**

Deux carboxyméthyl chitosanes d'origine animale, à usage pharmaceutique ou médical et implantables ou injectables ont pu être obtenus (CC-1 et CC-2 du Tableau 2a).

Deux autres carboxyméthyl chitosanes ont été testés :

- Un CC obtenu par carboxyméthylation de chitine d'origine animale (CC-3),
- Un CC obtenu par carboxyméthylation de chitosane d'origine animale, le chitosane ayant été préparé par déacétylation d'une chitine d'origine animale (CC-4).

Les carboxymethyl chitosanes (CC) ont été caractérisés par RMN du carbone 13 pour confirmer leur identité et déterminer les valeurs de DA (degré d'acétylation) et DS (degré de substitution par le groupe carboxyméthyle), avec une marge d'erreur estimée à environ $\pm$ 10% (Tableau 2a). On caractérise la charge électrostatique du polymère en mesurant son potentiel zeta à une concentration de 0,05% (m/m) sur une gamme de pH allant de pH 8 à pH 4, et on rapporte la valeur du potentiel zeta à pH 7,5 (Tableau 2a). On observe que la valeur du potentiel zeta à pH 7,5 est relativement bien corrélée à la valeur du DS déterminés par RMN du carbone 13. Par exemple le polymère CC-3 est plus substitué que le polymère CC-4 par le groupe anionique carboxyméthyl (sous forme carboxylate à pH 7,5) et présente un DA similaire, par conséquent sa charge globale négative est plus importante (-28mV vs. -11mV à pH 7,5).

On observe que les polymères CC-1 et CC-2 ne sont pas bien solubles dans la gamme de pH physiologique (pH 6,5 à 7,5). On ne peut pas évaluer leur immunocompatibilité à l'aide du modèle de poche à air chez la souris. Les polymères CC-3 et CC-4 sont bien solubles sur toute la gamme de pH large, de 1 à 9.

| Tableau 2a - Caractéristiques des carboxyméthyl chitosanes d'origine animale et de leurs formulations | | | | | |
|---|---|---|---|---|---|
| No (concentration) | DA* (mol % ) | DS* (mol %) | Gamme de solubilité du polymère | Stérilisabilité de la formulation par autoclave | Potentiel zeta à pH 7,5 (mV) |
| CC-1 (2%) | 8 | 124 | Insoluble entre pH 3,6 et 8,1 | / | / |
| CC-2 (2%) | / | / | Insoluble à pH inférieur à 6,6 | / | / |
| CC-3 (2%) | 71 | 149 | Soluble à tout pH | Non | -28 |
| CC-4 (1,5%) | 69 | 24 | Soluble à tout pH | Oui | -11 |
| *les valeurs de DA et DS des polymères carboxyméthyl chitosane sont déterminées par RMN du carbone 13 en phase solide. | | | | | |

Les formulations de CC-3 (2%) et CC-4 (1,5%) ont alors été préparées selon la même méthode que celle de l'Exemple 1, conditionnées en seringue en verre puis stérilisées par autoclave selon un cycle standard de 15 minutes à 121°C (Tableau 2a). On constate que la formulation à 2% du polymère CC-3 ne résiste pas à la stérilisation finale par autoclave, comme mis en évidence par une perte de sa viscosité intrinsèque d'environ 60% traduisant l'hydrolyse du polymère, ainsi que par une perte de 98% de son module de conservation G'. Alternativement à la stérilisation finale par autoclave, la formulation de CC-3 est alors filtrée avant d'être conditionnée en seringue afin de produire une formulation pouvant être évaluée à l'aide du modèle de poche à air chez la souris.

Les formulations de CC-3 (2%, filtrée) et CC-4 (1,5%, stérilisée par autoclave) sont contrôlées pour vérifier que le niveau d'endotoxines et la charge microbiologiques sont inférieurs à 20 EU/mL et 100 cfu/g, puis on évalue leur immunocompatibilité à l'aide du modèle de poche à air chez la souris (Tableau 2b).

| Tableau 2b - Immunocompatibilité des formulations de carboxyméthyl chitosane d'origine animale à l'aide du modèle de poche à air chez la souris, 24 heures après injection* | | | | |
|---|---|---|---|---|
| No | Nbe de globules blancs (x10$^6$ cellules/mL) | Conc. en IL1-beta (x10$^{-9}$g/mL)** | Conc. en KC/CXCL1 (x10$^{-9}$g/mL) | Conc. en TNF-alpha (x10$^{-9}$ g/mL)** |
| CC-3 | 0,7 | <3 | 49 | < 125 |

(suite)

| Tableau 2b - Immunocompatibilité des formulations de carboxyméthyl chitosane d'origine animale à l'aide du modèle de poche à air chez la souris, 24 heures après injection* | | | | |
|---|---|---|---|---|
| No | Nbe de globules blancs (x10$^6$ cellules/mL) | Conc. en IL1-beta (x10$^{-9}$g/mL)** | Conc. en KC/CXCL1 (x10$^{-9}$g/mL) | Conc. en TNF-alpha (x10$^{-9}$ g/mL)** |
| CC-4 | 1,0 | 5 | 115 | 184 |
| Contrôles | | | | |
| Contrôle négatif (salin) | 0,2 | 17 | 23 | 272 |
| Contrôle positif (1% carraghénane) | 33,2 | 28 | > 700 | 767 |
| Synvisc® (Hylan GF20) | 0,7 | 4 | 46 | < 125 |
| *valeur moyenne sur 3 sujets ; **limite de détection : 3.10$^{-9}$ g/mL pour IL1-beta et 125.10$^{-9}$ g/mL pour TNF-alpha. | | | | |

La formulation de CC-4 entraîne une réaction non négligeable, caractérisée par une concentration élevée des deux marqueurs KC/CXCL1 (activation des neutrophiles) et TNF-alpha dans l'infiltrat, simultanément. Le marqueur TNF-alpha n'a pas été détecté après injection des formulations de CSS de l'Exemple 2 ni de Synvisc® (Hylan GF-20). Or, il n'est pas désiré d'induire une réaction caractérisée par le marqueur TNF-alpha. Comme il est désiré de disposer d'une bonne immunocompatibilité dans le cas des applications thérapeutiques pour lesquelles la formulation serait en contact avec des tissus fragilisés par la pathologie, comme par exemple le syndrome de l'œil sec, des lésions de cornée ou des inflammations articulaires, et d'éviter l'activation des neutrophiles et de TNF-alpha, le carboxyméthyl chitosane d'origine animale CC-4 n'est pas approprié.

La formulation de CC-3 induit une réaction plus faible, caractérisée par un nombre de globules blancs et une concentration en marqueur KC/CXCL1 modérée, similaires aux niveaux du produit comparateur Synvisc® mais plus élevée que celle du contrôle négatif (salin). La formulation de CC-3 semble donc moins immunoréactive que les formulations de CSS et que la formulation de CC-4. Cependant la formulation de CC-3 présente le désavantage d'une concentration en marqueur KC/CXCL1 qui pourrait se révéler inadaptée pour une méthode de traitement thérapeutique où le sujet nécessite une très bonne immunocompatibilité et de subir une forte hydrolyse des chaînes macromoléculaires sous l'effet de la chaleur, ce qui rend impossible sa stérilisation par autoclave lors d'une étape finale en fin de procédé de production, c'est-à-dire après remplissage de la seringue par la formulation.

**Exemple 3 - Carboxymethyl chitosanes d'origine fongique**

Afin d'obtenir les carboxyméthyl chitosanes des Tableaux 3a et 3b au départ de chitine ou de chitosane d'origine fongique, on met en œuvre les réactions suivantes :

- Réacétylation puis carboxyméthylation de chitosane fongique de masse moléculaire « ultra-high » (CC-5 du Tableau 3a) ;
- Carboxyméthylation puis réacétylation de chitosane fongique de masse moléculaire « ultra-high » (CC-5 à CC-9) ;
- Carboxyméthylation de chitosane fongique de masse moléculaire « ultra-high » (CC-11) ;
- Carboxyméthylation de chitine fongique (CC-10).

A titre d'exemple, la préparation du CC-8 se déroule de la manière suivante : 10g de chitosane « ultra-high » sont dispersés dans 220mL d'isopropanol et 68mL d'hydroxyde de sodium à 40% (m/v). 45g de l'agent alkylant acide monochloroacétique (MCA) sont dissous dans 45mL d'isopropanol, et ajoutés à la suspension de chitosane. La réaction est poursuivie pendant 16 heures à 25°C. Le polymère est récupéré par précipitation dans l'éthanol, puis purifié par des cycles de solubilisation/précipitation dans l'éthanol. Le précipité est séché dans une étuve ventilée. Une masse de 15g du précipité est dispersée dans l'eau, et 3,75 mL d'anhydride acétique y sont ajoutés. Après 30 minutes d'agitation à température ambiante, le pH du milieu est ajusté à 6, et 3,75 mL d'anhydride acide (AC) y sont ajoutés. Après 30 minutes d'agitation à température ambiante, le milieu est neutralisé et précipité dans l'éthanol. Le précipité ainsi obtenu est remis en solution puis de nouveau précipité. Le précipité final de carboxyméthyl chitosane (CC-8) est séché dans une étuve ventilée

Les paramètres de synthèse appliqués pour préparer les autres CC sont décrits dans les Tableaux 3a et 3b.

| Tableau 3a - Paramètres de synthèse de carboxyalkyle chitosane au départ de chitosane fongique | | | | | | |
|---|---|---|---|---|---|---|
| **Paramètres** | **CC-5** | **CC-6** | **CC-7** | **CC-8** | **CC-9** | **CC-11** |
| **Etape de carboxyméthylation** | | | | | | |
| Agent alkylant (MCA ou SCA)* | SCA | MCA | MCA | MCA | MCA | MCA |
| NaOH/chitosane (m/m) | 11 | 2,7 | 2,7 | 2,7 | 2,7 | 2,7 |
| Urée | Oui | Non | Non | Non | Non | Non |
| Agent alkylant/chitosane (m/m) | 11,2 | 9 | 9 | 4,5 | 4,5 | 6,8 |
| Isopropanol/agent alkylant (%, m/m) | 0% | 80% | 80% | 80% | 80% | 80% |
| Température (°C) | 15°C | 15°C | 25°c | 25°C | 35°C | 60°C |
| Durée (heures) | 22h | 16h | 16h | 16h | 4h | 1h |
| **Etape de réacétylation** | | | | | | |
| Avant ou après carboxyméthylation | avant | après | après | après | après | / |
| AC/chitosane (v/m) -Premier ajout -Deuxième ajout | 0,3 0,3 | 0,25 0,25 | 0,25 0,25 | 0,25 0,25 | 0,25 0,25 | / |
| Durée et température de réaction | 0,5 heure à 25°C | 0,5 heure à 25°C | 0,5 heure à 25°C | 0,5 heure à 25°C | 0,5 heure à 25°C | / |
| *MCA : acide monochloroacétique ; SCA : sel de sodium de l'acide monochloroacétique. | | | | | | |

| Tableau 3b - Paramètres de synthèse de carboxyalkyl chitosane au départ de chitine fongique | |
|---|---|
| **Paramètres** | **CC-10** |
| Chitine/soude/eau (m/m) | 1/11,4/28,5 |
| Durée et température de contact avec la soude | 16 heures à 4°C |
| Agent alkylant (MCA ou SCA) | MCA |
| Chitine/isopropanol/agent alkylant (m/m/m) | 1/28,5/4,5 |
|  |  |
| Durée et température de réaction | 16 heures à 25°C |

Les carboxyméthyl chitosanes (CC) d'origine fongique obtenus sont caractérisés par RMN du carbone 13 pour confirmer leur structure et déterminer la valeur du DA et du DS (Tableau 3c). En tenant compte de la déviation standard inhérente à la méthode de spectrométrie RMN du carbone 13 (environ $\pm$ 10%), on observe que la valeur du potentiel zeta à pH 7,5 est corrélée à la structure moléculaire, en particulier au DS.

| Tableau 3c - Caractéristiques des carboxyméthyl chitosanes d'origine fongique et de leurs formulations | | | | | |
|---|---|---|---|---|---|
| No (concentration) | DA*% | DS*% | Solubilité du polymère | Stérilisable par autoclave** | Potentiel zeta à pH et 7,5 (mV) |
| CC-5 (1,5%) | 67 | 18 | Soluble à tout pH | Oui | -12,5 |
| CC-6 (2%) | 71 | 42 | Soluble à tout pH | Oui | -15 |
| CC-7 (2%) | 57 | 70 | Soluble à tout pH | Oui | -18 |
| CC-8 (2%) | 50 | 80 | Soluble à tout pH | Oui | -26 |
| CC-9 (2%) | 41 | 101 | Soluble à pH > 3,1 | Oui | -22 |
| CC-10 (2%) | 46 | 167 | Soluble à tout pH | Oui | -24,5 |
| CC-11 (2%) | 23 | 130 | Soluble à pH > 3,5 | Oui | -26 |
| *DA et DS déterminés par 13C-RMN ; **considéré comme stérilisable si la perte de viscosité intrinsèque avant/après stérilisation est inférieure à 40%. | | | | | |

Les CC obtenus sont tous solubles sur une large gamme de pH, en particulier sur la gamme de pH de 4,0 à 8,5, sans opalescence.

Les formulations sont préparées à une concentration en CC de 1,5 ou 2%, conditionnées dans une seringue en verre, puis stérilisées par autoclave selon un cycle standard de 15 minutes à 121°C. Afin d'évaluer si les formulations sont stérilisables par autoclave, on mesure la viscosité intrinsèque des polymères avant et après autoclave par viscosimétrie capillaire, après dilution d'un facteur de 25 par un tampon phosphate. La perte en viscosité intrinsèque des CC est inférieure à 40%, ce qui traduit une résistance acceptable, contrairement à la formulation CC-3 de l'Exemple 2 (qui a subi une perte d'environ 60% de sa viscosité intrinsèque).

L'inspection visuelle des formulations stérilisées du Tableau 3c indique qu'elles sont transparentes et non opalescentes. Elles sont alors contrôlées pour vérifier que le niveau d'endotoxines et la charge microbiologiques sont inférieurs à 20 EU/mL et 100 cfu/g, puis on évalue leur immunocompatibilité à l'aide du modèle de poche à air chez la souris.

| Tableau 3d - Immunocompatibilité des formulations de carboxyméthyl chitosane d'origine fongique à l'aide du modèle de poche à air chez la souris, 24 heures après injection* | | | | |
|---|---|---|---|---|
| No | Nbe de globules blancs (x10$^6$ cellules/ mL) | Conc. en IL1-beta (x10$^{-9}$g/mL) | Conc. en KC/CXCL1 (x10$^{-9}$g/mL)** | Conc. en TNF-alpha (x10$^{-9}$g/mL)** |
| CC-5 | 1,9 | 6 | 28 | 391 |
| CC-6 | 17 | 12 | 20 | < 125 |
| CC-7 | 9,0 | 12 | 20 | < 125 |
| CC-8 | 2,6 | <3 | 20 | < 125 |
| CC-9 | 6,5 | 7 | 20 | < 125 |
| CC-10 | 2,4 | <3 | 16 | < 125 |
| Contrôles | | | | |
| Contrôle négatif (salin) | 0,2 | 17 | 23 | 272 |

(suite)

| Contrôles | | | | |
|---|---|---|---|---|
| Contrôle positif (1% carraghénane) | 33,2 | 28 | > 700 | 767 |
| Synvisc® (Hylan GF20) | 0,7 | 4 | 46 | < 125 |
| *valeur moyenne sur 3 sujets ; **limite de détection : 3.10$^{-9}$ g/mL pour IL1-beta et 125.10$^{-9}$ g/mL pour TNF-alpha. | | | | |

D'une manière générale, on constate une bonne immunocompatibilité des formulations de CC d'origine fongique, avec une atténuation voire une suppression de la réaction immune par rapport aux formulations de N-succinyl chitosane de l'Exemple 1 et aux formulations de carboxyméthyl chitosan d'origine animale de l'Exemple 2.

On constate qu'une faible concentration en marqueur KC/CXCL1 est induite par les formulations de CC d'origine fongique, au même niveau que celle du contrôle négatif (salin) et plus faible que pour le produit Synvisc®. Ceci traduit une activation des neutrophiles négligeable voire absente, contrairement à ce qui a été rapporté après injection des formulations de CSS (Exemple 1) et de CC d'origine animale (Exemple 2).

Par ailleurs, on constate que pour certaines des formulations de CC d'origine fongique (i.e. CC-8 et CC-10), les quatre paramètres de la réaction immune sont atténués ou supprimés simultanément, c'est-à-dire à la fois le nombre total de globules blancs, le marqueur IL1-beta, le marqueur KC-CXCL1 et le marqueur TNF-alpha. Ceci ne se produit pas avec les formulations de CSS ni avec les formulations de CC d'origine animale.

Il apparaît que pour les CC d'origine fongique, les formulations qui présentent cette atténuation simultanée des quatre paramètres sont celles dont la charge négative est la plus élevée (e.g. -26mv pour CC-8 et -24,5mV pour CC-10 à pH 7,5).

On observe que les formulations de CC-4 d'origine animale de l'Exemple 2 et de CC-5 d'origine fongique faiblement substitués par le groupe carboxyalkyl entraînent une certaine activation du marqueur TNF-alpha, et que par contre seule la formulation de CC-4 d'origine animale entraîne une sécrétion non négligeable du marqueur KC/CXCL1. On observe également que la formulation de CC-10 (d'origine fongique) n'a pas entraîné d'activation des neutrophiles, contrairement à la formulation de CC-3 d'origine animale de l'Exemple 2.

**Exemple 4 - Evaluation de la tolérance locale intra-articulaire de formulations de carboxyméthyl chitosan d'origine fongique à l'aide d'un modèle ovin**

Des formulations à 2% de deux carboxyméthyl chitosane d'origine fongique (CC-8 et CC-10) de l'Exemple 3 sont préparées pour évaluer leur potentiel pour une utilisation par injection intra-articulaire, à l'aide d'un modèle ovin. Un volume de 2mL des deux formulations est administré chez la brebis, et la réaction locale induite par leur injection intra-articulaire est évaluée durant une période de 2 semaines. Les effets d'une deuxième injection de la formulation CC-8 dans la même articulation, 1 mois après la date de la première injection, ont également été évalués. Le produit Synvisc® est administré de la même manière, avec un volume de 1 mL ou de 2 mL.

Les signes cliniques sont suivis quotidiennement par palpation de l'articulation et observation de la boiterie pendant une durée de 15 jours et jugés selon un score semi-quantitatif de 0 (pas de signal) à un score maximum de 3 pour l'effusion et 5 pour la boiterie. Le résultat clinique global sur la période de 15 jours est rapporté en termes d'incidence par score (Tableau 4), ainsi que la caractérisation du fluide synovial réalisé au jour 15.

| Tableau 4-Evaluation de la tolérance locale de formulations de carboxyméthyl chitosan d'origine fongique et de Synvisc® à l'aide d'un modèle ovin par injection intra-articulaire | | | |
|---|---|---|---|
| No formulation Volume (N : nbe d'animaux) | Incidence de l'effusion sur 15 jours (score 0 à 3) | Incidence de la boiterie sur 15 jours (score 0 à 5) | Caractérisation du fluide synovial ponctionné au jour 15 |
| CC-8 Première injection de 2mL (N = 4) | Score 0 : 100% Scores 1 à 3 : 0% | Score 0 : 100% Scores 1 à 5 : 0% | Normal |
| CC-8 Deuxième injection de 2mL (N = 2) | Score 0 : 1 00% Scores 1 à 3 : 0% | Score 0 : 1 00% Scores 1 à 5 : 0% | Normal |

(suite)

| No formulation Volume (N : nbe d'animaux) | Incidence de l'effusion sur 15 jours (score 0 à 3) | Incidence de la boiterie sur 15 jours (score 0 à 5) | Caractérisation du fluide synovial ponctionné au jour 15 |
|---|---|---|---|
| **Tableau 4**-Evaluation de la tolérance locale de formulations de carboxyméthyl chitosan d'origine fongique et de Synvisc® à l'aide d'un modèle ovin par injection intra-articulaire | | | |
| CC-10 | Score 0 : 1 00% | Score 0 : 1 00% | Normal |
| **No formulation Volume (N : nbe d'animaux)** | **Incidence de l'effusion sur 15 jours (score 0 à 3)** | **Incidence de la boiterie sur 15 jours (score 0 à 5)** | **Caractérisation du fluide synovial ponctionné au jour 15** |
| Une injection de 2mL (N = 4) | Scores 1 à 3 : 0% | Scores 1 à 5 : 0% | |
| Synvisc® (Hylan GF-20) N = 8, dont : -une injection de 1mL (N = 4), -une injection de 2mL (N = 4) | Score 0 : 62,5% Score 1 : 37,5% Scores 2 à 3 : 0% | Score 0 : 62,5% Score 1 : 37,5% Scores 2 à 5 : 0% | Normal |

**Exemple 5 - Evaluation de la tolérance locale de formulations de carboxyméthyl chitosane d'origine fongique après injection intradermique chez le lapin**

Des formulations à pH et osmolalité physiologiques de trois carboxyméthyl chitosanes d'origine fongique sont préparées en vue d'évaluer leur potentiel pour l'administration intradermique, à l'aide d'un modèle lapin (Tableau 5a). Les CC-12, CC-13, CC-14 et CC-15 d'origine fongique sont préparés par carboxyméthylation puis réacétylation selon la méthode générale décrite à l'Exemple 3 pour le CC-8, en modulant les paramètres de synthèse pour faire varier le DA et le DS (Tableau 5a). CC-13 et CC-14 présentent un DS faible de 41% et 51% respectivement, et un DA élevé de 74% et 69% respectivement.

Les formulations sont conditionnées dans une seringue en verre de 1 mL, puis stérilisées par autoclave selon un cycle standard (15 minutes à 121°C). Les trois formulations résistent à la stérilisation par autoclave, la perte de viscosité intrinsèque étant inférieure à 40%. Enfin, les formulations sont contrôlées pour vérifier que le niveau d'endotoxines bactériennes est inférieur à 40 EU/mL et la charge microbiologique inférieure à 100 cfu/mL.

Un volume de 200 μL de formulation est administré par injection intradermique chez le lapin via une aiguille de diamètre 27 Gauge (aiguille de diamètre très faible), selon un protocole compliant avec la norme ISO10993 (partie 10) pour l'évaluation de l'irritation primaire induite par un implant intradermique. Un nombre de six lapins reçoivent chacun trois injections de chaque formulation. On injecte également un produit de comblement cutané commercial à base d'acide hyaluronique réticulé, Juvéderm® Voluma (Allergan), via une aiguille de diamètre 27 Gauge. Les signaux macroscopiques d'irritation cutanée sont rapportés pour tous les animaux et les sites injectés à 12, 24 et 48 heures en vue de déterminer le score d'irritation primaire, en évaluant l'apparition éventuelle d'érythème, d'eschare, d'œdème et d'induration sur une échelle semi-quantitative, selon un score de 0 (pas de signal) à 4 (signal maximum). Le score d'irritation primaire de chaque produit est déterminé de la manière suivante : la moyenne des scores d'érythème des 18 sites injectés à 24h, 48h et 72h est additionnée. On calcule de la même façon la somme des moyennes du score d'œdème. On additionne les 2 sommes (érythème et œdème), puis on divise par 54 pour obtenir le score moyen d'irritation primaire. On procède de la même façon avec le produit comparateur. Les scores d'irritation sont rapportés dans le Tableau 5b. On observe que les formulations de CC n'ont entraîné aucun signe d'œdème et peu de signes d'érythème, de score inférieur à ceux induits par Juvéderm® Voluma dans tous les cas. On conclut que les formulations sont non-irritantes et moins irritantes que Juvéderm® Voluma.

**Tableau 5a** - Caractéristiques des carboxyméthyl chitosanes d'origine fongique et de leurs formulations

| No (concentration) | DA* (mol %) | DS* (mol %) | Solubilité du polymère | Stérilisable par autoclave** | Potentiel zeta à pH 7,5 (mV) |
|---|---|---|---|---|---|
| CC-12 (3%) | 41 | 50 | Insoluble entre pH 3,1 et 6.6 | Oui | -17 |
| CC-13 (3%) | 74 | 45 | Soluble à tout pH | Oui | -20 |
| CC-14 (3%) | 69 | 51 | Soluble au-dessus de pH 3,6 | Oui | -17*** |
| CC-15 (2%) | 55 | 100 | Soluble au-dessus de pH 3,1 | Oui | -27,5 |

*déterminés par 13C-RMN ; **considéré comme stérilisable si la perte de viscosité intrinsèque avant/après stérilisation par autoclave est inférieure ou égale à 40% ; ***valeur estimée à partir de la régression polynomiale de la courbe potentiel zeta à pH 7,5 en fonction du DS ($\pm$ 20%).

**Tableau 5b** - Evaluation du score d'irritation primaire de formulations de carboxyméthyl chitosane et de Juvéderm® Voluma après injection intradermique chez le lapin

| No (concentration) | Score d'irritation primaire à 72 heures Score total (et score moyen) |
|---|---|
| CC-12 (3%) | 0,11 (0,002) |
| CC-13 (3%) | 0,22 (0,004) |
| CC-13 (3%) | 0,17 (0,003) |
| CC-14 (2%) | 0 (0) |
| Juvéderm® Voluma | 2,67 (0,049) |

La période d'observation est ensuite prolongée jusqu'à 2 semaines après injection, avec une évaluation des signaux cliniques aux jours 5, 7, 9, 11 et 14. En vue de réaliser une analyse histologique éventuelle, 2 animaux sont sacrifiés au Jour 3, Jour 7 et Jour 14 après injection.

Les scores macroscopiques sont rapportés dans le Tableau 5c. On observe que les quatre formulations présentent une excellente tolérance locale sur une période de 2 semaines après injection intradermique, avec des scores inférieurs à ceux observés avec le produit Juvéderm® Voluma pour tous les signes macroscopiques, et aucun signe d'eschare ni d'œdème. Les formulations de CC-12 et de CC-13 présentent quelques signes d'érythème de score faible (1 maximum) qui ont disparu au Jour 11. La formulation de CC-13 présente quelques signes d'érythème de score maximum 2, avec une incidence de 33% au Jour 9, qui ont également disparu au Jour 11 et n'entraînent pas d'effet délétère sur le tissu.

**Tableau 5c** - Evaluation de la tolérance locale de formulations de carboxyméthyl chitosane après injection intradermique chez le lapin (2 semaines) (exprimée en termes d'incidence des scores)

| No (concentration) | Délai (nbe sites)* | Jour 3 (18) | Jour 5 (12) | Jour 7 (12) | Jour 9 (6) | Jour 11 (6) | Jour 14 (6) |
|---|---|---|---|---|---|---|---|
| **Score d'érythème** | | | | | | | |
| CC-12 (3%) | Score 0-1 | 100% | 100% | 100% | 100% | 100% | 100% |
| | Score 2-4 | 0% | 0% | 0% | 0% | 0% | 0% |
| CC-13 (3%) | Score 0-1 | 100% | 100% | 83% | 67% | 100% | 100% |
| | Score 2-4 | 0% | 0% | 17% | 33% | 0% | 0% |
| CC-14 (3%) | Score 0-1 | 100% | 100% | 100% | 100% | 100% | 100% |

(suite)

**Tableau 5c** - Evaluation de la tolérance locale de formulations de carboxyméthyl chitosane après injection intradermique chez le lapin (2 semaines) (exprimée en termes d'incidence des scores)

| No (concentration) | Délai (nbe sites)* | Jour 3 (18) | Jour 5 (12) | Jour 7 (12) | Jour 9 (6) | Jour 11 (6) | Jour 14 (6) |
|---|---|---|---|---|---|---|---|
| **Score d'érythème** | | | | | | | |
| | Score 2-4 | 0% | 0% | 0% | 0% | 0% | 0% |
| CC-15 (2%) | Score 0-1 | 100% | 100% | 100% | 100% | 100% | 100% |
| | Score 2-4 | 0% | 0% | 0% | 0% | 0% | 0% |
| Juvéderm® Voluma | Score 0-1 | 6% | 17% | 17% | 17% | 33% | 33% |
| | Score 2-4 | 94% | 83% | 83% | 83% | 67% | 67% |
| **Scores d'eschare et œdème** | | | | | | | |
| CC-12 (3%) | Score 0-1 | 100% | 100% | 100% | 100% | 100% | 100% |
| | Score 2-4 | 0% | 0% | 0% | 0% | 0% | 0% |
| CC-13 (3%) | Score 0-1 | 100% | 100% | 83% | 67% | 100% | 100% |
| | Score 2-4 | 0% | 0% | 17% | 33% | 0% | 0% |
| CC-14 (3%) | Score 0-1 | 100% | 100% | 100% | 100% | 100% | 100% |
| | Score 2-4 | 0% | 0% | 0% | 0% | 0% | 0% |
| CC-15 (2%) | Score 0-1 | 100% | 100% | 100% | 100% | 100% | 100% |
| | Score 2-4 | 0% | 0% | 0% | 0% | 0% | 0% |
| Juvéderm® Voluma | Score 0-1 | 100% | 100% | 100% | 100% | 100% | 100% |
| | Score 2-4 | 0% | 0% | 0% | 0% | 0% | 0% |

*nombre de sites évalués macroscopiquement à chaque délai après injection. Par ailleurs, les quatre compositions (CC-12, CC-13, CC-14 et CC-15) peuvent être facilement injectées au travers d'une aiguille fine de 27 Gauge, adéquate pour l'administration intradermique.

**Exemple 6 - Susceptibilité à la dégradation enzymatique *in vitro***

Dans cet exemple, on compare la vitesse à laquelle une formulation est dégradée en présence d'un mélange des deux enzymes lysozyme et hyaluronidase présents dans les fluides biologiques (par exemple le fluide synovial, les larmes ou le fluide interstitiel des tissus conjonctifs). L'enzyme lysozyme est généralement reconnu pour sa capacité à hydrolyser les biomatériaux à base de chitosane.

Un carboxyméthyl chitosane fongique CC-16 est préparé de la même manière que le CC-8 de l'Exemple 3, en modulant les paramètres de synthèse pour ajuster le DA et le DS. Des formulations à 2% de CC-16, de CC-8 (Exemple 3) et de CC-10 (Exemple 3) sont préparées dans un tampon phosphate avec 3,5% de sorbitol. On évalue également une formulation à 2% du CC-3 d'origine animale (Exemple 2), et deux produits de viscosupplémentation commerciaux à base d'acide hyaluronique non réticulé (HA-1 et HA-2).

La formulation est diluée d'un facteur 25 dans un tampon phosphate. La solution est ensuite agitée pendant 12 heures et un mélange d'enzymes lysozyme et hyaluronidase est ajouté à la solution diluée, aux doses de 184 unités/mL et 2,6 unités/mL, respectivement. La mesure de viscosité intrinsèque se fait à intervalle régulier, à l'aide d'un viscosimètre capillaire automatique I-Visc (Lauda) muni d'un capillaire de type Ubbelohde (modèle 0a). On calcule alors le temps de demi-vie de chaque formulation, c'est-à-dire le temps nécessaire pour que la viscosité intrinsèque du polymère atteigne la moitié de sa valeur initiale (Tableau 6).

**Tableau 6** - Temps de demi-vie des formulations en présence d'un mélange d'enzymes lysozyme/hyaluronidase à 37°C

| | CC-8 (2%) | CC-10 (2%) | CC-16 (2%) | CC-3 (2%) | HA-1 | HA-2 |
|---|---|---|---|---|---|---|
| **DA / DS (mol %)*** | 50 / 80 | 46 / 167 | 67 / 115 | 71 / 149 | / | / |

(suite)

| Tableau 6 - Temps de demi-vie des formulations en présence d'un mélange d'enzymes lysozyme/hyaluronidase à 37°C | | | | | | |
|---|---|---|---|---|---|---|
| | CC-8 (2%) | CC-10 (2%) | CC-16 (2%) | CC-3 (2%) | HA-1 | HA-2 |
| Temps de demi-vie (minutes) | 1200 min | 958 min | 612 min | 88 min | 73 min | 11 min |
| *déterminés par RMN du carbone 13 en phase solide. | | | | | | |

On comprend des résultats que les formulations de carboxyméthyl chitosane sont hydrolysées par le mélange lysozyme/hyaluronidase, et que l'on peut moduler la cinétique d'hydrolyse via la structure moléculaire du carboxyméthyl chitosane. Ceci permet d'ajuster la durée de résidence du produit en fonction de l'application thérapeutique visée. On déduit également que les carboxyméthyl chitosanes d'origine fongique sont moins rapidement dégradés que le carboxyméthyl chitosane d'origine animale (CC-3) et que les deux produits commerciaux à base d'acide hyaluronique.

**Exemple 7 - Impact de la chaleur**

Dans cet exemple, on place des seringues contenant les formulations dans une étuve à température contrôlée à 60°C pendant une durée de 11 jours, ce qui permet d'évaluer leur résistance à la chaleur à une température plus élevée qu'une température de stockage habituelle. A chaque délai, on prélève une seringue et on mesure la viscosité intrinsèque du polymère selon la méthode décrite à l'Exemple 6. On rapporte le ratio entre la viscosité au délai et la viscosité initiale (en % de la viscosité initiale).

Deux carboxyméthyl chitosanes d'origine fongique, CC-17 et CC-18, sont préparés de la même manière que le CC-8 de l'Exemple 3, en modulant les paramètres de synthèse pour ajuster le DA et le DS, et caractérisés par RMN du carbone 13 (Tableau 7). Ils sont formulés à une concentration de 2% en présence d'un sucre réducteur, le sorbitol ou le mannitol, respectivement. On évalue également une formulation à 2% du CC-3 d'origine animale de l'Exemple 2 (avec 3,5% de sorbitol), ainsi que le produit de viscosupplémentation commercial à base d'acide hyaluronique non réticulé de l'Exemple 6 (HA-2).

| Tableau 7 - Evolution de la viscosité intrinsèque des formulations conservées à 60°C (en % de la viscosité intrinsèque initiale) | | | | |
|---|---|---|---|---|
| No (concentration, sucre réducteur) | DA / DS (mol %)* | Jour 3 | Jour 7 | Jour 11 |
| CC-17 (2%, 3,5% sorbitol) | 71 / 49 | 96% | 95% | 91% |
| CC-18 (2%, 3,5% mannitol) | 71 / 49 | 100% | 92% | 91% |
| CC-3 (2%, 3,5% sorbitol) | 71 /149 | 84% | 80% | 70% |
| HA-2 | / | 94% | 92% | 81% |
| *déterminés par RMN du carbone 13 en phase solide | | | | |

On conclut que les formulations de carboxyméthyl chitosane d'origine fongique sont résistantes à la chaleur, et moins sensibles que la formulation d'origine animale (CC-3) et qu'un produit commercial à base d'acide hyaluronique.

**Exemple 8 - Procédés de réduction de la charge microbiologique de carboxymethyl chitosane**

Un carboxyméthyl chitosane d'origine fongique, CC-19, est produit selon la méthode utilisée pour préparer le CC-8 de l'Exemple 3, au départ d'un chitosane « ultra-high ». Ses caractéristiques sont les suivantes : DA 67% et DS 115% (mesurés par RMN du carbone 13), soluble à tout pH, transparente, non opalescente. Le potentiel zeta à pH 7,5 de la formulation est estimé à -27mV (à partir de la régression polynomiale de la courbe du potentiel zeta en fonction du DS). On prépare une formulation de CC-19 à 2% dans un tampon phosphate avec 3,5% de sorbitol, comme décrit à l'Exemple 3. Ses caractéristiques sont les suivantes : pH 7,3 et osmolalité 279 mOsm/kg. Afin de tester leur faisabilité et l'impact sur les caractéristiques finales de la formulation, on met en œuvre deux procédés connus pour pouvoir réduire la charge microbiologique de formulations aqueuses, et on compare la viscosité intrinsèque de la formulation (diluée d'un facteur de 25), à l'aide d'un viscosimètre capillaire I-Visc (Lauda, capillaire 0a) et son indice de réfraction mesuré à l'aide d'un réfractomètre HI88713 (Hanna) avant et après procédé (Tableau 8). Les deux procédés sont les suivants :

- Autoclave : la formulation est mise en seringue en verre, puis la seringue est autoclavée selon un procédé standard (15 minutes à 121°C) ;

- Filtration : un volume de 300mL de formulation est filtré à l'aide d'un filtre de porosité $0,2\mu m$ (Preflow capsule filter, Pall).

Le procédé de filtration sur filtre de $0,2\mu m$ se déroule de manière adéquate, avec une pression constante de 2 bars et un débit constant d'environ 6 litres par heure.

| Tableau 8 - Impact des procédés de filtration et d'autoclave sur la formulation de CC-19 à 2% | | |
|---|---|---|
| **Indicateur** | **Procédé de filtration ($0,2\mu m$)** | **Procédé par autoclave (15 min 121°C)** |
| **Différence de viscosité intrinsèque (%)** | 14% de réduction de la viscosité initiale | 20% de réduction de viscosité intrinsèque |
| **Différence d'indice de réfraction** | Pas de différence | Pas de différence |

On conclut que les deux procédés ont un impact faible en termes de réduction de matière (indice de réfraction) et de dégradation du polymère (viscosité intrinsèque). Ils sont donc applicables industriellement pour obtenir des dispositifs médicaux et des produits pharmaceutiques à base des formulations de carboxyméthyl chitosane.

**Exemple 9 - Capacité lubrifiante des formulations de carboxyméthyl chitosan d'origine fongique, *in vitro***

Cet exemple vise à illustrer la capacité lubrifiante de formulations de deux carboxyméthyl chitosanes d'origine fongique, CC-8 de l'Exemple 3 (2% et 1%) et CC-19 de l'Exemple 8 (2%), en vue d'un usage éventuel comme viscosupplément intra-articulaire ou comme goutte ophtalmique pour la surface oculaire. La capacité lubrifiante est évaluée à l'aide d'un modèle *in vitro* qui permet de d'évaluer la réduction des frictions entre deux surfaces. La formulation de CC-3 (2%) d'origine animale de l'Exemple 2 et des produits commerciaux à base d'acide hyaluronique sont également caractérisés :

- Viscosuppléments intra-articulaires : Synvisc® (Sanofi) et deux viscosuppléments à base d'acide hyaluronique non réticulé, HA-2 des Exemples 6 et 7) et HA-3.
- Gouttes ophtalmiques : deux produits à base d'acide hyaluronique non réticulé (HA-4 et HA-5)

Par ailleurs, on prélève le fluide synovial du genou d'un patient souffrant d'ostéoarthrose, avant une intervention chirurgicale pour la pose d'une prothèse de genou. Le fluide est conservé à -20°C, puis mis à 25°C avant l'analyse du coefficient de friction.

La mesure du coefficient de friction est réalisée selon la méthode suivante. Deux disques à base d'un biomatériau de type polyacrylate utilisé pour la fabrication de lentilles intraoculaires hydrophobes (tel que décrit dans le brevet EP 1830898 de diamètre 16,15 mm sont préalablement hydratés par immersion dans l'eau à 60°C pendant environ 2 heures, puis fixés sur les géométries supérieure et inférieure d'un rhéomètre DHR-2 (TA Instruments). Un volume d'environ 100 $\mu$L du fluide à tester est placé sur le disque inferieur, puis la géométrie supérieure est descendue jusqu'au contact entre les deux disques, jusqu'à une force normale imposée de 5 Newton. Les mesures du coefficient de friction sont réalisées à 25°C pendant une durée de 150 secondes, à force normale constante (5N), fréquence d'oscillation de 1,256 rad/s et angle de déformation d'environ 0,05 radian, selon un protocole adapté du protocole décrit par Waller et al. (in : J Rheumatol 39, 7, 1473, 2012). L'option « respect du point zéro de départ du mouvement oscillatoire » est activée. A chaque point de mesure, on enregistre la valeur du torque, puis on calcule le coefficient de friction (COF) selon la formule : COF = torque / (1/3 x diamètre du disque x force normale). Pour chaque formulation, la mesure est répliquée 5 fois. On rapporte la valeur du coefficient de friction par extrapolation de l'ordonnée à l'origine au départ de chaque courbe COF en fonction du temps (COFO, Tableau 7).

| Tableau 9 - Capacité lubrifiante des formulations de carboxyméthyl chitosan d'origine fongique | | | |
|---|---|---|---|
| No (concentration) | Coefficient de friction ($COF_0$) | | |
| | Mesure 1 | Mesure 2 | Mesure 3 |
| Fluide synovial d'un patient arthrosique | 20 | 40 | 12 |
| CC-8 (2%) | 1,2 | 1,7 | 1,3 |
| CC-8 (1%) | 2,4 | 3,7 | 3,9 |
| CC-19 (2%) | 4,0 | 3,2 | 3,0 |
| CC-3 (2%) | 6,2 | 6,1 | 6,0 |
| Synvisc® | 3,2 | 3,1 | 3,8 |
| HA-2 | 6,7 | 6,3 | 6,6 |
| HA-3 | 5,3 | 5,6 | 4,5 |
| HA-4 | 6,8 | 6,7 | 6,0 |
| HA-5 | 29,4 | 13,3 | 26,1 |

En présence des formulations de carboxyméthyl chitosane à 2% et 1%, le coefficient de friction est faible, du même ordre de grandeur voire plus faible que les produits commerciaux à usage intra-articulaire et ophtalmique, et significativement plus faible que celui d'un fluide synovial arthrosique, dans les conditions de mesure. On en déduit que les formulations de carboxyméthyl chitosane présentent le potentiel d'agir comme lubrifiant en réduisant les frictions entre deux surfaces, par exemple les surfaces de cartilage d'une articulation après injection intra-articulaire ou la surface oculaire après instillation sous forme de gouttes. Les formulations des CC d'origine fongique sont plus efficaces pour réduire une réduction des frictions que la formulation de CC-3 d'origine animale.

**Exemple 10 - Utilisation d'aiguilles fines pour l'administration d'une formulation de carboxyméthyl chitosan d'origine fongique par voie intradermique**

Cet exemple vise à montrer qu'une formulation de 2% de carboxyméthyl chitosane d'origine fongique peut être facilement injectée dans le derme, en particulier à l'aide d'aiguilles très fines conçues pour l'injection dans les couches superficielles du derme. Le test consiste à mesurer la force nécessaire pour éjecter le produit hors de la seringue équipée d'une aiguille, à une vitesse d'éjection donnée, à l'aide d'un banc de compression. On considère de manière empirique que l'injection du produit est aisée et confortable pour le médecin et le patient lorsque la force d'éjection mesurée par ce test est inférieure à 50 Newton, et que l'éjection se fait de manière régulière et sans à-coups. Il est avantageux de pouvoir administrer le produit à l'aide des aiguilles de diamètre inférieur à 0.3mm (30G) afin de minimiser la douleur et le saignement à l'injection ainsi que le risque ultérieur d'hématomes et de rougeur cutanée.

Un carboxyméthyl chitosane d'origine fongique de référence CC-20 est produit selon la méthode générale du CC-8 de l'Exemple 3, au départ d'un chitosane de type « ultra-high », avec les modifications suivantes : pour 10g de chitosane, on utilise 228ml d'isopropanol, 57ml d'hydroxyde de sodium à 40% et 47g de MCA. La réaction est menée à 35°C pendant 23 heures. Pour 15g de carboxyméthyl chitosan intermédiaire, on utilise 7,5ml d'anhydride acétique à chaque ajout, et 3 cycles supplémentaires de purification sont appliqués avant séchage et récupération du carboxyméthyl chitosane final. Les caractéristiques de CC-20 sont les suivantes : DA 53% et DS 85% (déterminés par RMN du carbone 13), soluble dans l'eau à tout pH (selon la méthode précédemment décrite), formant une solution transparente et non opalescente, de potentiel zeta à pH 7,5 estimé à -24mV (à partir de la régression polynomiale de la courbe du potentiel zeta en fonction du DS).

On prépare une formulation de CC-20 à 2% (m/m) comme décrit à l'Exemple 3. La formulation est conditionnée dans une seringue en verre de 1mL (BD-Medical) sur laquelle l'aiguille est adaptée. A l'aide d'un banc de compression MultiTest 2.5-i (Mecmesin) équipé avec une cellule de compression de 100N, on mesure la force nécessaire pour l'éjection du produit en appliquant une vitesse d'éjection constante de 80mm/min. La force maximale tolérée par l'équipement est d'environ 70 Newton. On teste les aiguilles suivantes : 30G, 32G, 33G et Invisible Needle™ (TSK Laboratory), dont les dimensions (diamètre extérieur x longueur) sont rapportées au Tableau 10.

A titre de comparaison, des produits commerciaux à base d'acide hyaluronique non réticulé (références HA-6, HA-7) et réticulé (HA-8), indiqués pour la réjuvénation cutanée par voie intradermique, sont évalués selon la même méthode, dans leur seringue d'origine. Les résultats sont rapportés au Tableau 10.

| Tableau 10-Force d'éjection (en Newton) d'une formulation de carboxyméthyl chitosan d'origine fongique et de produits commerciaux de réjuvénation cutanée au travers d'aiguilles intradermiques (vitesse 80mm/min) | | | | |
|---|---|---|---|---|
| Référence de l'aiguille | TSK PRC-30013I | TSK PRE-32009 | TSK PRE-3304 | TSK LDS-02009 |
| Dimensions | 30G 0,3 x 13mm | 32G 0,26 x 9mm | 33G 0,24 x 4mm | Invisible 0,2 x 9mm |
| CC-20 (2%) | 12 N | 26 N | 27 N | 39 N |
| HA-6 | / | 30 N | 30 N | 44 N |
| Référence de l'aiguille | TSK PRC-30013I | TSK PRE-32009 | TSK PRE-3304 | TSK LDS-02009 |
| Dimensions | 30G 0,3 x 13mm | 32G 0,26 x 9mm | 33G 0,24 x 4mm | Invisible 0,2 x 9mm |
| HA-7 | 23 N | 29 N | 32 N | 40 N |
| HA-8 | 22 N | 45 N | 46 N | 67 N |

On conclut de cet exemple que, comme les produits de réjuvénation à base d'acide hyaluronique non réticulé, la formulation de CC-20 est facilement injectable à l'aide d'aiguilles fines de diamètre inférieur à 0,3mm. On note que la force d'éjection mesurée est même systématiquement inférieure à celle des produits de réjuvénation à base d'acide hyaluronique non réticulé. Le produit de réjuvénation à base d'acide hyaluronique réticulé nécessite une force systématiquement plus élevée, et il dépasse la limite acceptable avec l'aiguille du plus petit diamètre (0,2mm, Invisible Needle™). Par ailleurs, ces résultats ont été confirmés par des médecins spécialisés, via un test qualitatif consistant à injecter la formulation de CC-20 et les produits de réjuvénation dans la peau d'une patte de cochon.

## Exemple 11 - Formulations de carboxyméthyl chitosan d'origine fongique de faible concentration en vue d'une indication comme gouttes ophthalmiques

Cet exemple vise à montrer qu'une solution de carboxymethyl chitosan d'origine fongique de faible concentration peut être appliquée pour la préparation de gouttes ophtalmiques en vue de réduire les symptômes des troubles de la surface oculaire ou de la protéger. En plus de caractéristiques physiologiques, c'est-à-dire préférentiellement un pH autour de 7,2 et une osmolalité autour de 200 mOsm/kg, les gouttes ophtalmiques doivent de préférence présenter les propriétés physico-chimiques suivantes : indice de réfraction faible (proche de 1,33) et capacité à minimiser les frictions entre la surface oculaire et la conjonctive et les paupières (capacité lubrifiante). Enfin, son profil rhéologique est tel que le produit n'est pas trop fluide pour pouvoir se déposer sur l'œil, mais qu'il s'étale de manière homogène et qu'il n'est pas collant. Son profil rhéologique doit aussi être tel que que le battement des paupières est aisé, sans accumulation, soit une viscosité faible à fort taux de cisaillement. En particulier, Orobia et al. considèrent que la viscosité lors du mouvement oculaire (hors battement des paupières)) est avantageusement supérieure à 10 mPa.s, par exemple autour de 20 mPa.s (in : Clinical Ophthalmology 12, 453, 2018). Il faut noter ici que la valeur de la viscosité est susceptible de varier selon la méthode de mesure, en particulier l'équipement, le mode de mesure et les paramètres, la température et le taux de cisaillement appliqué au produit testé.

Un carboxyméthyl chitosane d'origine fongique de référence CC-21 est produit selon la même méthode que le CC-20 de l'Exemple 10. On compare la structure moléculaire de CC-20 et CC-21 sous forme acide, par spectrométrie infra-rouge à transformée de Fourrier à l'aide d'un spectromètre Nicolet iS5 équipé d'un ID7-ATR (Thermo Scientifics), selon la méthode décrite par Chen et al. (Carbohydrate Polymers 53, 355, 2003). On détermine que leur structure moléculaire est identique à 99% dans la région de longueur d'onde de 1200 à 1800 $cm^{-1}$, et par conséquent que leurs DA et DS sont similaires.

On prépare deux formulations du carboxyméthyl chitosane CC-21, de concentration 0,7% et 0,4% (m/m), dans un tampon phosphate avec du glycérol. On ajuste le pH à 7,2 ± 0,2 et l'osmolalité à 200 ± 20 mOsm/kg, puis les formulations sont filtrées, puis leur capacité lubrifiante et leur profil de viscosité sont caractérisés selon les méthodes décrites ci-dessous. Des gouttes ophtalmiques à base d'acide hyaluronique (HA) de composition variable, disponibles commercialement, sont caractérisées selon les mêmes méthodes (HA-5 à HA-9). Les résultats sont rapportés au Tableau 11b.

Méthode de mesure du coefficient de friction (adaptée aux gouttes ophtalmiques)

La capacité à lubrifier, c'est-à-dire de réduire les frictions entre deux surfaces en contact, est évaluée par la mesure du coefficient de friction entre deux disques en biomatériau polyacrylate identiques à ceux de l'Exemple 9. Les deux disques sont fixés sur les géométries supérieure et inférieure d'un rhéomètre DHR-2 (TA Instruments), un volume

d'environ 100 μL du produit à tester est placé sur le disque inférieur, puis la géométrie supérieure est descendue jusqu'au contact entre les deux disques, jusqu'à une force normale imposée de 5 Newton. La mesure du coefficient de friction est réalisée à 25°C pendant une durée de 60 secondes, à force normale constante (5 N), fréquence d'oscillation de 6 rad/s et angle de déformation d'environ 1.71 radian, selon un protocole adapté de Waller et al. (J Rheumatol 39, 7, 1473, 2012) et des paramètres mimant les conditions cinématiques de clignement des paupières décrites par Kwon et al. (J Royal Society Interface 10, 2, 2013). L'option « respect du point zéro de départ du mouvement oscillatoire » est activée. A chaque point de mesure, on enregistre la valeur du torque, puis on calcule le coefficient de friction (COF) selon la formule : COF = torque / (1/3 x diamètre du disque x force normale). On détermine la valeur moyenne du COF entre 0 et 60 s. On calcule ainsi le coefficient de friction moyen et l'écart-type de 5 mesures consécutives. La valeur minimum du COF est de 52 dans le cas où les deux surfaces ne sont pas en contact. La limite supérieure de COF correspond au cas où les deux disques ne sont plus en mouvement l'un par rapport à l'autre.

Etant donné que la valeur de COF est variable d'une série à l'autre, il faut caractériser les produits à comparer dans une même série, à l'aide des mêmes disques et dans un ordre aléatoire. On utilise alors une échelle de scores relatifs pour classer les produits testés dans une même série, du plus efficace (score 1) au moins efficace (score 3), selon les critères du Tableau 11a. Les limites « $COF_A$ » et « $COF_B$ » sont définies par le COF de deux gouttes ophtalmiques commerciales prises comme référence à chaque série : gouttes A (composées de 0,15% HA et 0,25% carbomer) et gouttes B (0,15% HA). Selon cette échelle, il est avantageux que le score de réduction des frictions soit de 1 ou 2, et de préférence de 1, pour un usage comme goutte lubrifiante de la surface oculaire. Pour les produits de score 3, la capacité lubrifiante n'est pas satisfaisante et la variation sur la mesure est importante.

| Tableau 11a-Echelle de scores de lubrification (réduction des frictions) | |
|---|---|
| Score | Limites de COF |
| 1 | $\leq 1,1 \times COF_A$ |
| 2 | $> 1,1 \times COF_A$ et $\leq 1,1 \times COF_B$ |
| 3 | $> 1,1 \times COF_B$ |

Méthodes de mesure de la viscosité en fonction du cisaillement

Simmons et al. ont déterminé que le taux de cisaillement lors des mouvements de l'œil ouvert est d'environ 10s-1 et le taux de cisaillement lors du battement des paupières est d'environ 10000s-1 (in : Clinical Ophthalmology 11, 1637, 2017). On sélectionne la méthode de mesure rhéométrique en fonction de la gamme de taux cisaillement à étudier et en tenant compte que les produits sont des solutions fluides de faible viscosité :

- Gamme de cisaillement correspondant au mouvement oculaire. On mesure la viscosité en mode rotationnel avec un test de balayage en écoulement à l'aide d'un rhéomètre DHR-2 à régulation de stress (TA Instruments) équipé d'un Peltier plat et une géométrie de type « cône » de 60mm de diamètre et un angle de troncature de 2°. Le produit est équilibré pendant 1 minute à 37°C, la température étant contrôlée par le Peltier. Pour éviter l'évaporation, le système est équipé d'un piège à solvant et une couverture métallique. Ensuite, le test de balayage en écoulement est démarré et la viscosité est mesurée en fonction du taux de cisaillement, de $0,001s^{-1}$ à $100s^{-1}$. La valeur de viscosité à 10 s$^{-1}$ est rapportée.
- Gamme de cisaillement correspondant au battement des paupières. On mesure la viscosité en mode rotationnel avec un test de balayage en écoulement à l'aide d'un rhéomètre DHR-3 à régulation de stress (TA Instruments) équipé d'une géométrie de type « Couette à double gap » et d'un Peltier en cylindre concentrique, et équipé d'un piège à solvant et une couverture métallique. Le produit est équilibré pendant 1 minute à 37°C, puis on applique un taux de cisaillement variable de $0,001s^{-1}$ à $10000s^{-1}$. La valeur de viscosité à $10000s^{-1}$ est alors comparée par rapport à la valeur à 10s$^{-1}$.

| Tableau 11b- Caractéristiques de formulations de carboxyméthyl chitosan d'origine fongique de faible concentration et de gouttes ophtalmiques à base d'acide hyaluronique | | | | |
|---|---|---|---|---|
| Référence | Indice de réfraction | Viscosité | | Score de réduction des frictions |
| | | à 10s$^{-1}$ (mPa.s) | à 10000s$^{-1}$ (mPa.s) | |
| Tampon phosphate avec glycérol | 1,3348 | N/A | N/A | Non mesurable |
| CC-21 0,7% | 1,3358 | 27 | Inférieure à la viscosité à 10s-1 | 1 |
| CC-21 0,4% | 1,3353 | 10 | | 1 |
| HA-5 | 1,3343 | 6 | Inférieure à la viscosité à 10s-1 | 2 |
| HA-10 | 1,3386 | 3 | | 2 |
| HA-11 | 1,3350 | 6 | | 2 |
| HA-12 | 1,3346 | 50 | | 2 |

Les résultats confirment que les deux formulations de carboxyméthyl chitosane fongique CC-21 répondent au cahier des charges de gouttes ophtalmiques : elles présentent un indice de réfraction faible, un coefficient de friction satisfaisant (score 1, c'est-à-dire aussi efficace que les gouttes commerciales les plus lubrifiantes), une viscosité 10 à 30 mPa.s dans les conditions de mouvement oculaire et une viscosité plus faible dans les conditions de battement des paupières. Sans le carboxyméthyl chitosane, le tampon phosphate supplémenté en glycérol ne permet pas de répondre au cahier des charges des gouttes ophtalmiques. Enfin, on constate que la viscosité peut être diminuée en réduisant la concentration en carboxyméthyl chitosane (par exemple de 0,7 à 0,4%) sans altérer la capacité à réduire les frictions (score 1).

**Revendications**

1. Carboxyalkyl chitosane d'origine fongique présentant des unités glucosamine, des unités N-acétyl-glucosamine et des unités glucosamine substituées par un groupe carboxyalkyl, ledit carboxyalkyl chitosane présentant un degré de substitution par un groupe carboxyalkyl supérieur à 50%, exprimé en nombre de mole du substituant par rapport au nombre de mole d'unités totales.

2. Carboxyalkyl chitosane selon la revendication 1, **caractérisé en ce que** le degré de substitution par un groupe carboxyalkyl est inférieur à 200%, exprimé en nombre de mole du substituant par rapport au nombre de mole d'unités totales.

3. Carboxyalkyl chitosane selon l'une quelconque des revendications 1 à 2, **caractérisé en ce que** le chitosane est issu du mycélium d'un champignon du type *Ascomycète,* et en particulier d'*Aspergillus niger,* et/ou d'un champignon *Basidiomycète,* et en particulier *Lentinula edodes* (shiitake) et/ou *Agaricus bisporus* (champignon de Paris), de préférence, le chitosane est issu *d'Agaricus bisporus.*

4. Carboxyalkyl chitosane selon l'une quelconque des revendications 1 à 3, **caractérisé en ce que** le carboxyalkyl chitosane présente un degré d'acétylation de 40% à 80%, exprimé en nombre de mole unités N-acétyl- glucosamine par rapport au nombre de mole d'unités totales.

5. Carboxyalkyl chitosane selon l'une quelconque des revendications 1 à 4, **caractérisé en ce que** le carboxyalkyl chitosane est réacétylé.

6. Carboxyalkyl chitosane selon l'une quelconque des revendications 1 à 5, **caractérisé en ce qu'**il est stérilisé.

7. Composition **caractérisée en ce qu'**elle comprend au moins un carboxyalkyl chitosane défini selon l'une quelconque des revendications 1 à 6.

8. Composition injectable **caractérisée en ce qu'**elle comprend au moins un carboxyalkyl chitosane défini selon l'une quelconque des revendications 1 à 6.

9. Composition pharmaceutique **caractérisée en ce qu'**elle comprend au moins un carboxyalkyl chitosane défini selon l'une quelconque des revendications 1 à 6.

10. Composition, selon la revendication 8 ou 9, **caractérisée en ce qu'**elle est utilisée comme pharmaceutique injectable, implantable ou apte à l'instillation, ou dispositif médical injectable ou implantable ou apte à l'instillation.

11. Composition selon la revendication 10, pour une utilisation dans une méthode de traitement thérapeutique, par exemple comprenant l'instillation ou l'injection par voie sous-cutanée, intradermique, oculaire, intraoculaire, ou intra-articulaire, de ladite composition, par exemple pour la réparation ou le comblement d'au moins un tissu corporel nécessitant une réparation ou un comblement.

12. Composition, selon la revendication 11, **caractérisée en ce que** le tissu corporel est choisi parmi les tissus appartenant aux cordes vocales, muscles, ligaments, tendons, cartilages, organes sexuels, os, articulations, yeux, derme, ou l'une quelconque de leurs combinaisons, et plus particulièrement aux joints articulaires.

13. Composition selon la revendication 11 ou 12, pour son utilisation dans une méthode de traitement d'une arthrose, ou la réparation d'un défaut de cartilage, par exemple par injection dans le fluide synovial ou après mélange avec le sang et implantation dans le cartilage.

14. Dispositif médical, par exemple implant médical, **caractérisé en ce qu'**il comprend ou consiste en une composition telle que définie à l'une quelconque des revendications 7 à 13.

15. Procédé de préparation d'une composition comprenant un carboxyalkyl chitosane défini selon l'une quelconque des revendications 7 à 13, ledit procédé comprenant :

- la dissolution d'un carboxyalkyl chitosane défini selon l'une quelconque des revendications 1 à 8 dans une solution aqueuse, de préférence de l'eau, éventuellement tamponnée, de préférence à pH compris entre 6,2 et 8,5 et de préférence entre 6,5 et 7,5 ;
- l'ajustement éventuel du pH à un pH désiré, en général au pH physiologique pour l'application visée, par exemple par ajout d'un agent tamponnant, d'une base ou d'un acide ;
- l'ajout éventuel d'autres excipients, comme par exemple un sucre réducteur, par exemple le sorbitol ou le mannitol ;
- l'ajustement éventuel de l'osmolalité de la composition.

**Patentansprüche**

1. Carboxyalkylchitosan pilzlichen Ursprungs, das Glucosamineinheiten, N-Acetylglucosamineinheiten und durch eine Carboxyalkylgruppe substituierte Glucosamineinheiten aufweist, wobei das Carboxyalkylchitosan einen Substitutionsgrad durch eine Carboxyalkylgruppe von mehr als 50% aufweist, ausgedrückt als Molzahl des Substituenten im Verhältnis zur Molzahl der gesamten Einheiten.

2. Carboxyalkylchitosan nach Anspruch 1 , **dadurch gekennzeichnet, dass** der Substitutionsgrad durch eine Carboxyalkylgruppe kleiner als 200% ist, ausgedrückt als Molzahl des Substituenten bezogen auf die Molzahl der gesamten Einheiten.

3. Carboxyalkylchitosan nach einem der Ansprüche 1 bis 2, **dadurch gekennzeichnet, dass** das Chitosan aus dem Myzel eines Pilzes des *Ascomyceten* Typs und insbesondere eines *Aspergillus niger* und/oder eines *Basidiomyceten* Pilzes und insbesondere *Lentinula edodes* (Shiitake) und/oder *Agaricus bisporus* (Zuchtchampignon) hervorgeht, vorzugsweise geht das Chitosan aus dem *Agaricus bisporus* hervor.

4. Carboxyalkylchitosan nach einem der Ansprüche 1 bis 3, **dadurch gekennzeichnet, dass** das Carboxyalkylchitosan einen Acetylierungsgrad von 40% bis 80% aufweist, ausgedrückt als Molzahl der N-Acetylglucosamin-Einheiten bezogen auf die Molzahl der gesamten Einheiten.

5. Carboxyalkylchitosan nach einem der Ansprüche 1 bis 4, **dadurch gekennzeichnet, dass** das Carboxyalkylchitosan reacetyliert ist.

6. Carboxyalkylchitosan nach einem der Ansprüche 1 bis 5, **dadurch gekennzeichnet, dass** es sterilisiert ist.

7. Verbindung, **dadurch gekennzeichnet, dass** sie mindestens ein Carboxyalkylchitosan nach einem der Ansprüche 1 bis 6 enthält.

8. Injizierbare Verbindung, **dadurch gekennzeichnet, dass** sie mindestens ein Carboxyalkylchitosan umfasst, das nach einem der Ansprüche 1 bis 6 definiert ist.

9. Pharmazeutische Verbindung, **dadurch gekennzeichnet, dass** sie mindestens ein Carboxyalkylchitosan enthält, das nach einem der Ansprüche 1 bis 6 definiert ist.

10. Verbindung nach Anspruch 8 oder 9, **dadurch gekennzeichnet, dass** sie als eine injizierbare, implantierbare oder instillierbare pharmazeutische Verbindung oder als ein injizierbares oder implantierbares oder zur Instillation geeignetes Medizinprodukt verwendet wird.

11. Verbindung nach Anspruch 10 zur Verwendung in einem therapeutischen Behandlungsverfahren, beispielsweise die Instillation oder Injektion der Verbindung auf subkutanem, intradermalem, okularem, intraokularem oder intraartikulärem Weg beispielsweise zur Wiederherstellung oder Verstärkung von mindestens einem Körpergewebe, das eine Wiederherstellung oder eine Verstärkung benötigt, umfassend.

12. Verbindung für die Verwendung nach Anspruch 11, **dadurch gekennzeichnet, dass** das Körpergewebe aus den Geweben ausgewählt ist, die zu den Stimmbändern, Muskeln, Bändern, Sehnen, Knorpeln, Geschlechtsorganen, Knochen, Gelenken, Augen, Dermis oder einer beliebigen ihrer Kombinationen und insbesondere zu Gelenken gehören.

13. Verbindung für die Verwendung nach Anspruch 11 oder 12, **dadurch gekennzeichnet, dass** die Behandlung ein Verfahren zur Behandlung von Arthrose oder zur Behebung eines Knorpeldefekts ist, beispielsweise durch Injektion in die Gelenkflüssigkeit oder nach Mischen mit dem Blut und Implantation in den Knorpel.

14. Medizinische Vorrichtung, beispielsweise ein medizinisches Implantat, **dadurch gekennzeichnet, dass** sie eine Verbindung nach einem der Ansprüche 7 bis 13 umfasst oder daraus besteht.

15. Verfahren zur Herstellung einer Verbindung, die ein Carboxyalkylchitosan nach einem der Ansprüche 7 bis 13 enthält, wobei das Verfahren umfasst:

   Auflösen eines Carboxyalkylchitosans, definiert nach einem der Ansprüche 1 bis 8, in einer wässrigen Lösung, vorzugsweise Wasser, gegebenenfalls gepuffert, vorzugsweise bei einem pH-Wert zwischen 6,2 und 8,5 und bevorzugt zwischen 6,5 und 7,5;
   gegebenenfalls Einstellen des pH-Wertes auf einen gewünschten pH-Wert, im allgemeinen auf einen für die beabsichtigte Anwendung physiologischen pH-Wert, beispielsweise durch Zugabe eines Puffers, einer Base oder einer Säure;
   gegebenenfalls Zugeben anderer Hilfsstoffe, wie beispielsweise eines reduzierenden Zuckers, beispielsweise Sorbit oder Mannit;
   gegebenenfalls Einstellen der Osmolalität der Verbindung.

**Claims**

1. A carboxyalkyl chitosan of fungal origin having glucosamine units, N-acetyl-glucosamine units and glucosamine units substituted by a carboxyalkyl group, the said carboxyalkyl chitosan having a degree of substitution by a carboxyalkyl group that is greater than 50%, expressed as the number of moles of the substituent in relation to the number of moles of total units.

2. A carboxyalkyl chitosan according to claim 1, **characterised in that** the degree of substitution by a carboxyalkyl group is less than 200%, expressed in number of moles of the substituent in relation to the number of moles of total units.

3. A carboxyalkyl chitosan according to any one of claims 1 to 2, **characterised in that** the chitosan is derived from the mycelium of a fungus of the type *Ascomycetes,* and in particular from *Aspergillus niger,* and/or from a fungus *Basidiomycetes,* and in particular *Lentinula edodes* (shiitake) and/or *Agaricus bisporus* (button mushroom), preferably, the chitosan is derived from *Agaricus bisporus.*

4.  A carboxyalkyl chitosan according to any one of claims 1 to 3, **characterised in that** the carboxyalkyl chitosan has a degree of acetylation ranging from 40% to 80%, expressed in number of moles of N-acetyl-glucosamine units in relation to the number of moles of total units.

5.  A carboxyalkyl chitosan according to any one of claims 1 to 4, **characterised in that** the carboxyalkyl chitosan is reacetylated.

6.  A carboxyalkyl chitosan according to any one of claims 1 to 5, **characterised in that** it is sterilised.

7.  A composition **characterised in that** it comprises at least one carboxyalkyl chitosan defined according to any one of claims 1 to 6.

8.  An injectable composition **characterised in that** it comprises at least one carboxyalkyl chitosan defined according to any one of claims 1 to 6.

9.  A pharmaceutical composition, **characterised in that** it comprises at least one carboxyalkyl chitosan defined according to any one of claims 1 to 6.

10. A composition according to claim 8 or 9, **characterised in that** it is used as a pharmaceutical composition that is injectable, implantable or suitable for instillation, or a medical device that is injectable, implantable or suitable for instillation.

11. A composition according to claim 10, for use in a therapeutic treatment method, for example comprising the instillation or the injection of the said composition, via a subcutaneous, intradermal, ocular, intraocular, or intra-articular route, for example for the repair or filling in of at least one body tissue requiring repair or filling in.

12. A composition according to claim 11, **characterised in that** the body tissue is selected from the tissues belonging to the vocal cords, muscles, ligaments, tendons, cartilages, sexual organs, bones, joints, eyes, dermis, epidermis, or any one of the combinations thereof, and more particularly belonging to the articular joints.

13. A composition according to claim 11 or 12, for the use thereof in a treatment method for treating osteoarthritis, or in the repair of a cartilage defect, for example by means of injection into the synovial fluid or after mixing with the blood and implantation in the cartilage.

14. A medical device, for example a medical implant, **characterised in that** it comprises or consists of a composition as defined in any one of claims 8 to 14.

15. A preparation method for preparing a composition comprising a carboxyalkyl chitosan defined according to any one of claims 7 to 13, the said method including:

    - the dissolution of a carboxyalkyl chitosan defined according to any one of claims 1 to 8 in an aqueous solution, preferably water, optionally buffered, preferably having a pH comprised between 6.2 and 8.5, and preferably between 6.5 and 7.5;
    - the optional adjustment of the pH to a desired pH, in general to the physiological pH for the targetted application, for example by adding a buffering agent, an acid or a base;
    - the optional addition of other excipients, such as for example a reducing sugar, for example sorbitol or mannitol;
    - the optional adjustment of the final osmolality of the composition.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2016016463 A
- WO 2016016464 A
- CN 1431229 A
- CN 106474569
- WO 03068824 A
- EP 1483299 A
- US 7556946 B
- WO 2017009335 A
- WO 2017009346 A
- EP 3016663 A
- EP 1830898 A

**Littérature non-brevet citée dans la description**

- **RINAUDO et al.** *Int J Biol Macromol,* 1993, vol. 15, 281
- **LIU et al.** *Carb Polym,* 2016, vol. 137, 600
- *Clin Orthop Relat Res,* 1988, vol. 235, 289-95
- *Biochem Biophys Res Comm,* 2012, vol. 422, 455-461
- *J Bone Joint Surg Br,* 1959, vol. 41-B (2), 388-400
- *Acta Orthop Scand,* 1969, 634-641
- *Clin Rheumatol,* 2006, vol. 25, 886-888
- **SEGWICK et al.** *J Pathol,* 1983, vol. 141, 483
- **SIN et al.** *Ann Rheum Dis,* 1986, vol. 45, 873
- **DAWSON et al.** *Int J Tiss Reac,* 1991, vol. 8, 171
- **LITTLE et al.** *Osteoarthritis Cartilage,* 2010, vol. 18, S80
- **EDOUARD et al.** *Phys Ther Sport,* 2013, vol. 14, 116
- **MCCOY et al.** *Vet Pathol,* 2015, vol. 52, 803
- **FRASER et al.** *Semin Arthritis Rheum,* 1993, (9), 22
- **SHAFFORD et al.** *Vet Anaesth Analg,* 2004, vol. 31, 20
- **KALER et al.** *Vet J,* 2009, vol. 180, 189
- **WALLER et al.** *J Rheumatol,* 2012, vol. 39 (7), 1473
- **OROBIA et al.** la viscosité lors du mouvement oculaire (hors battement des paupières)) est avantageusement supérieure à 10 mPa.s, par exemple autour de 20 mPa.s. *Clinical Ophthalmology,* 2018, vol. 12, 453
- **CHEN et al.** *Carbohydrate Polymers,* 2003, vol. 53, 355
- **KWON et al.** *J Royal Society Interface,* 2013, vol. 10, 2
- **SIMMONS et al.** le taux de cisaillement lors des mouvements de l'œil ouvert est d'environ 10s-1 et le taux de cisaillement lors du battement des paupières est d'environ 10000s-1. *Clinical Ophthalmology,* 2017, vol. 11, 1637